# EUROPEAN PATENT APPLICATION

(11) **EP 3 685 830 A1**
(43) Date of publication of application: **29.07.2020**
(21) Application number: 19219435.5
(22) Date of filing: 20.05.2016
(51) Int. Cl.: A61K 31/00, A61K 38/00, A61K 48/00, A61P 35/00, A61P 31/00, A61P 37/00

(54) **METHODS AND PHARMACEUTICAL COMPOSITION FOR MODULATION POLARIZATION AND ACTIVATION OF MACROPHAGES**

(30) Priority: 20.05.2015 EP 15305759
(62) Divisional of application: 16725818.5
(71) Applicant: INSTITUT NATIONAL DE LA SANTE ET DE LA RECHERCHE MEDICALE - INSERM, 75013 Paris (FR); Universite Paris-Sud, 91400 Orsay (FR); Institut Gustave Roussy (IGR), 94800 Villejuif (FR); Institut Pasteur, 75015 Paris (FR); ASSISTANCE PUBLIQUE, HOPITAUX DE PARIS, 75004 Paris (FR); UNIVERSITE PARIS DESCARTES, 75006 Paris (FR); Université Paris Diderot Paris 7, 75013 Paris (FR); Université Pierre et Marie Curie (Paris 6), 75005 Paris (FR); Istituto Nazionale per le Malattie Infettive "Lazzaro Spallanzani" I.R.C.C.S, 00149 Roma (IT)
(72) Inventor: PERFETTINI, Jean-Luc, 94805 VILLEJUIF (FR); PAOLETTI, Audrey, 94805 VILLEJUIF (FR); GOUGEON, Marie-Lise, 75015 PARIS (FR); KROEMER, Guido, 75006 PARIS (FR); PIACENTINI, Mauro, 00149 ROMA (IT)
(74) Representative: Regimbeau

(57) **Abstract**

The present invention relates to methods and pharmaceutical composition for modulation polarization and activation of macrophages. In particular, the present invention relates to methods for modulating macrophage M1/M2 polarization in a subject in need thereof comprising administering to the subject a therapeutically effective amount of P2Y2 receptor agonists or antagonists.

SEQUENCE LISTING

<110>   Institut Gustave Roussy (IGR)
        Université Paris Diderot (Paris 7)
        Universite Paris-Sud
        INSERM - Institut National de la Santé et de la Recherche Médicale
        Institut Pasteur
        Assistance Publique-Hôpitaux de Paris
        Université Paris Descartes
        Université Pierre et Marie Curie (Paris 6)
        Istituto Nazionale per le Malattie Infettive "Lazzaro Spallanzani"
I.R.C.C.S

<120>  METHODS AND PHARMACEUTICAL COMPOSITION FOR MODULATION
       POLARIZATION AND ACTIVATION OF MACROPHAGES

<130>  B75777 D38737

<160>  15

<170>  PatentIn version 3.5

<210>  1
<211>  1036
<212>  PRT
<213>  Homo sapiens

<400>  1

<210>  2
<211>  19
<212>  RNA
<213>  Artificial Sequence

<220>
<223>  siRNA

<400>  2
ggaucaaacu acucuguga                                                    19

<210>  3
<211>  19
<212>  RNA
<213>  Artificial Sequence

<220>
<223>  siRNA

<400>  3
ugcaagaucu cucagcaaa                                                    19

<210>  4
<211>  19

<212>  RNA
<213>  Artificial Sequence

<220>
<223>  siRNA

<400>  4
uucaauaaau ucuugaggu                                                    19


<210>  5
<211>  29
<212>  DNA
<213>  Artificial Sequence

<220>
<223>  shRNA

<400>  5
atgttccacc tggctgtgtc tgatgcact                                         29


<210>  6
<211>  24
<212>  DNA
<213>  Artificial Sequence

<220>
<223>  shRNA

<400>  6
aaacccaggg ctgccttgga aaag                                              24


<210>  7
<211>  24
<212>  DNA
<213>  Artificial Sequence

<220>
<223>  shRNA

<400>  7
aaacccaggg ctgccttgga aaag                                              24


<210>  8
<211>  24
<212>  DNA
<213>  Artificial Sequence

<220>
<223>  shRNA

<400>  8
aaacccaggg ctgccttgga aaag                                              24


<210>  9
<211>  24
<212>  DNA
<213>  Artificial Sequence

<220>
<223>  shRNA

<400>  9
aaacccaggg ctgccttgga aaag                                              24


<210>  10
<211>  24
<212>  DNA
<213>  Artificial Sequence

<220>
<223>  shRNA

<400>  10
aaacccaggg ctgccttgga aaag                                              24


<210>  11
<211>  24
<212>  DNA
<213>  Artificial Sequence

<220>
<223>  shRNA

<400>  11
aaacccaggg ctgccttgga aaag                                              24


<210>  12
<211>  19
<212>  RNA
<213>  Artificial Sequence

<220>
<223>  siRNA

<400>  12
ugccuagggc caagcgcaa                                                    19


<210>  13
<211>  19
<212>  RNA
<213>  Artificial Sequence

<220>
<223>  siRNA

<400>  13
uaacuggagc uccgauuua                                                    19


<210>  14
<211>  19
<212>  RNA
<213>  Artificial Sequence

<220>
<223>  siRNA

<400>  14
ucucaggagu agucucaua                                                    19

<210>  15
<211>  19
<212>  RNA
<213>  Artificial Sequence

<220>
<223>  siRNA

<400>  15
agucaucguu uguguguau                                                    19

## Description

### FIELD OF THE INVENTION:

The present invention relates to methods and pharmaceutical composition for modulation polarization and activation of macrophages.

### BACKGROUND OF THE INVENTION:

Macrophages derived from monocyte precursors undergo specific differentiation depending on the local tissue environment. The various macrophage functions are linked to the type of receptor interaction on the macrophage and the presence of cytokines. Similar to the T helper type 1 and T helper type 2 (TH1-TH2) polarization, two distinct states of polarized activation for macrophages have been defined: the classically activated (M1) macrophage phenotype and the alternatively activated (M2) macrophage phenotype. Similar to T cells, there are some activating macrophages and some suppressive macrophages, therefore, macrophages should be defined based on their specific functional activities. Granulocyte macrophage colony stimulating factor (GM-CSF) and macrophage colony stimulating factor (M-CSF) are involved in the differentiation of monocytes to macrophages. Human GM-CSF can polarize monocytes towards the M1 macrophage subtype with a "pro-inflammatory" cytokine profile (e.g. TNF-alpha, IL-1beta, IL-6, IL-12 and IL-23), and treatment with M-CSF produces an "antiinflammatory" cytokine (e.g. IL-10, TGF-beta and IL-1ra) profile similar to M2 macrophages. Classically activated (M1) macrophages have the role of effector cells in TH1 cellular immune responses. The alternatively activated (M2) macrophages appear to be involved in immunosuppression and tissue repair. LPS and the TH1 cytokine IFN-gamma polarize macrophages towards the M1 phenotype which induces the macrophage to produce large amounts of IL-1beta, TNF, IL-12, and IL-23. This helps to drive antigen specific TH1 and TH17 cell inflammatory responses forward and thus participates to the clearance of invading microorganisms. The antimicrobial functions of M1 macrophages are linked to up-regulation of enzymes, such as inducible nitric oxide synthase (iNOS) that generates nitric oxide from L-arginine. The secretion of IL-6, IL-23, and IL-1beta are important factors in the induction and maintenance of Th17 cells. In some cases inflammatory responses can trigger tissue damage (toxic activity or reactive oxygen), resulting in an uncontrolled macrophage inflammatory response which could become pathogenic. For example, uncontrolled macrophage inflammatory response participates in the pathogenesis of inflammatory bowel disease (IBD). In contrast, exposure of macrophages to the TH2 cytokine IL-4 produces a M2 phenotype which induces the production of high levels of IL-10 and IL-1RA and low expression of IL-12. These cells reduce inflammation, are immunoregulators, promote tissue remodeling and tumor progression. It has recently been demonstrated that macrophages in vitro are capable of complete repolarization from M2 to M1, and change again in response to fluctuations in the cytokine environment. In particular, macrophages are important tumor-infiltrating cells and play pivotal roles in tumor growth and metastasis. In most solid tumors, the existence of macrophages is advantageous for tumor growth and metastasis. Recent studies indicate that tumor-associated macrophages (TAMs) show a M2 phenotype. These tumor-associated macrophages (TAM) produce interleukin IL-10 and transforming growth factor (TGF) β to suppress general antitumor immune responses. Meanwhile, TAMs promote tumor neo-angiogenesis by the secretion of pro-angiogenic factors and define the invasive microenvironment to facilitate tumor metastasis and dissemination. For these reasons, reducing the pool of M2 TAMs has been considered as a relevant approach to anti-cancer therapy.

### SUMMARY OF THE INVENTION:

The inventors have identified a new immune checkpoint capable of modulating polarization and activation of macrophages. Thus the present invention relates to methods and pharmaceutical composition for modulation polarization and activation of macrophages. In particular, the present invention is defined by the claims.

More precisely, the invention relates to:
1. A method of reducing macrophage M1 polarization in a subject in need thereof comprising administering to the subject a therapeutically effective amount of a P2Y2 receptor agonist.
2. A method of reducing the secretion of an inflammatory cytokine selected from the group consisting of IL-6, IL-23, and IL-1beta by macrophages in a subject in need thereof comprising administering to the subject a therapeutically effective amount of a P2Y2 receptor agonist.
3. A method of increasing M2 macrophages pool in a subject suffering from conditions associated with undesirable M1 polarization comprising administering to the subject a therapeutically effective amount of a P2Y2 receptor agonist.
4. A method of treating an inflammatory disease in a subject in need thereof comprising administering to the subject a therapeutically effective amount of a P2Y2 receptor agonist.
   In this method, said inflammatory disease is preferably selected from the group consisting of: sepsis, septicemia, pneumonia, septic shock, systemic inflammatory response syndrome (SIRS), Acute Respiratory Distress Syndrome (ARDS), acute lung injury, aspiration pneumanitis, infection, pancreatitis, bacteremia, peritonitis, abdominal abscess, inflammation due to trauma, inflammation due to surgery, chronic inflammatory disease, ischemia, ischemia-reperfusion injury of an organ or tissue, tissue damage due to disease, tissue damage due to chemotherapy or radiotherapy, and reactions to ingested, inhaled, infused, injected, or delivered substances, glomerulonephritis, bowel infection, opportunistic infections, and for subjects undergoing major surgery or dialysis, subjects who are immunocompromised, subjects on immunosuppressive agents, subjects with HIV/AIDS, subjects with suspected endocarditis, subjects with fever, subjects with fever of unknown origin, subjects with cystic fibrosis, subjects with diabetes mellitus, subjects with chronic renal failure, subjects with bronchiectasis, subjects with chronic obstructive lung disease, chronic bronchitis, emphysema, or asthma, subjects with febrile neutropenia, subjects with meningitis, subjects with septic arthritis, subjects with urinary tract infection, subjects with necrotizing fasciitis, subjects with other suspected Group A streptococcus infection, subjects who have had a splenectomy, subjects with recurrent or suspected enterococcus infection, other medical and surgical conditions associated with increased risk of infection, Gram positive sepsis, Gram negative sepsis, culture negative sepsis, fungal sepsis, meningococcemia, post-pump syndrome, cardiac stun syndrome, stroke, congestive heart failure, hepatitis, epiglotittis, E. coli 0157:H7, malaria, gas gangrene, toxic shock syndrome, pre-eclampsia, eclampsia, HELP syndrome, mycobacterial tuberculosis, Pneumocystic carinii, pneumonia, Leishmaniasis, hemolytic uremic syndrome/thrombotic thrombocytopenic purpura, Dengue hemorrhagic fever, pelvic inflammatory disease, Legionella, Lyme disease, Influenza A, Epstein-Barr virus, encephalitis, inflammatory diseases and autoimmunity including Rheumatoid arthritis, osteoarthritis, progressive systemic sclerosis, systemic lupus erythematosus, inflammatory bowel disease, idiopathic pulmonary fibrosis, sarcoidosis, hypersensitivity pneumonitis, systemic vasculitis, Wegener's granulomatosis, transplants including heart, liver, lung kidney bone marrow, graft-versus-host disease, transplant rejection, sickle cell anemia, nephrotic syndrome, toxicity of agents such as OKT3, cytokine therapy, cryoporin associated periodic syndromes and cirrhosis.
5. A method of treating an auto-immune disease in a subject in need thereof comprising administering to the subject a therapeutically effective amount of a P2Y2 receptor agonist.
   In this method, said autoimmune disease is preferably selected from the group consisting of Addison's Disease, Allergy, Alopecia Areata, Alzheimer's disease, Antineutrophil cytoplasmic antibodies (ANCA)-associated vasculitis, Ankylosing Spondylitis, Antiphospholipid Syndrome (Hughes Syndrome), arthritis, Asthma, Atherosclerosis, Atherosclerotic plaque, autoimmune disease (e.g., lupus, RA, MS, Graves' disease, etc.), Autoimmune Hemolytic Anemia, Autoimmune Hepatitis, Autoimmune inner ear disease, Autoimmune Lymphoproliferative syndrome, Autoimmune Myocarditis, Autoimmune Oophoritis, Autoimmune Orchitis, Azoospermia, Behcet's Disease, Berger's Disease, Bullous Pemphigoid, Cardiomyopathy, Cardiovascular disease, Celiac Sprue/Coeliac disease, Chronic Fatigue Immune Dysfunction Syndrome (CFIDS), Chronic idiopathic polyneuritis, Chronic Inflammatory Demyelinating, Polyradicalneuropathy (CIPD), Chronic relapsing polyneuropathy (Guillain-Barre syndrome), Churg-Strauss Syndrome (CSS), Cicatricial Pemphigoid, Cold Agglutinin Disease (CAD), chronic obstructive pulmonary disease (COPD), CREST syndrome, Crohn's disease, Dermatitis, Herpetiformus, Dermatomyositis, diabetes, Discoid Lupus, Eczema, Epidermolysis bullosa acquisita, Essential Mixed Cryoglobulinemia, Evan's Syndrome, Exopthalmos, Fibromyalgia, Goodpasture's Syndrome, Hashimoto's Thyroiditis, Idiopathic Pulmonary Fibrosis, Idiopathic Thrombocytopenia Purpura (ITP), IgA Nephropathy, immunoproliferative disease or disorder (e.g., psoriasis), Inflammatory bowel disease (IBD), including Crohn's disease and ulcerative colitis, Insulin Dependent Diabetes Mellitus (IDDM), Interstitial lung disease, juvenile diabetes, Juvenile Arthritis, juvenile idiopathic arthritis (JIA), Kawasaki's Disease, Lambert-Eaton Myasthenic Syndrome, Lichen Planus, lupus, Lupus Nephritis, Lymphoscytic Lypophisitis, Ménière's Disease, Miller Fish Syndrome/acute disseminated encephalomyeloradiculopathy, Mixed Connective Tissue Disease, Multiple Sclerosis (MS), muscular rheumatism, Myalgic encephalomyelitis (ME), Myasthenia Gravis, Ocular Inflammation, Pemphigus Foliaceus, Pemphigus Vulgaris, Pernicious Anaemia, Polyarteritis Nodosa, Polychondritis, Polyglandular Syndromes (Whitaker's syndrome), Polymyalgia Rheumatica, Polymyositis, Primary Agammaglobulinemia, Primary Biliary Cirrhosis/Autoimmune cholangiopathy, Psoriasis, Psoriatic arthritis, Raynaud's Phenomenon, Reiter's Syndrome/Reactive arthritis, Restenosis, Rheumatic Fever, rheumatic disease, Rheumatoid Arthritis, Sarcoidosis, Schmidt's syndrome, Scleroderma, Sjörgen's Syndrome, Stiff-Man Syndrome, Systemic Lupus Erythematosus (SLE), systemic scleroderma, Takayasu Arteritis, Temporal Arteritis/Giant Cell Arteritis, Thyroiditis, Type 1 diabetes, Type 2 diabetes, Ulcerative colitis, Uveitis, Vasculitis, Vitiligo, and Wegener's Granulomatosis.
6. A method for promoting secretion of an inflammatory cytokine selected from the group consisting of IL-1beta, TNF, IL-12, and IL-23 by macrophage in a subject in need thereof comprising administering to the subject a therapeutically effective amount of an inhibitor of P2Y2 receptor activity or expression.
7. A method of treating an infectious disease in a subject in need thereof comprising administering to the subject a therapeutically effective amount of an inhibitor of P2Y2 receptor activity or expression.
   In this method, the infectious disease is preferably a viral infection, a bacterial infection or a fungal infection.
8. A method of reducing macrophage M2 polarization in a subject in need thereof comprising administering to the subject a therapeutically effective amount of an inhibitor of P2Y2 receptor activity or expression.
9. A method of treating a condition associated with undesirable M2 macrophage polarization comprising administering to the subject a therapeutically effective amount of an inhibitor of P2Y2 receptor activity or expression.
   In this method, the condition associated with undesirable M2 macrophage polarization is preferably selected from the group consisting of cancer, especially metastatic cancer, progressive fibrotic diseases such as for example idiopathic pulmonary fibrosis (IPF), hepatic fibrosis systemic sclerosis, allergy and asthma, atherosclerosis and Alzheimer's disease.
   Said cancer is for example selected from the group consisting of cancer cells from the bladder, blood, bone, bone marrow, brain, breast, colon, esophagus, gastrointestinal tract, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous; adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; Paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; Sertoli cell carcinoma; Leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malignant melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia. In some embodiments, the method of the present invention is particularly suitable for the treatment of metastatic cancer to bone, wherein the metastatic cancer is breast, lung, renal, multiple myeloma, thyroid, prostate, adenocarcinoma, blood cell malignancies, including leukemia and lymphoma; head and neck cancers; gastrointestinal cancers, including esophageal cancer, stomach cancer, colon cancer, intestinal cancer, colorectal cancer, rectal cancer, pancreatic cancer, liver cancer, cancer of the bile duct or gall bladder; malignancies of the female genital tract, including ovarian carcinoma, uterine endometrial cancers, vaginal cancer, and cervical cancer; bladder cancer; brain cancer, including neuroblastoma; sarcoma, osteosarcoma; and skin cancer, including malignant melanoma or squamous cell cancer.
10. A method of treating an infectious disease in a subject in need thereof comprising administering to the subject a therapeutically effective amount of a LRR polypeptide or a nucleic acid molecule encoding thereof.

### DETAILED DESCRIPTION OF THE INVENTION:

Purinergic receptors and NLR proteins are major actors of innate immune responses. Despite extensive studies, the complex signaling network between these innate sensors is poorly understood. Here, the inventors show that the NLR family member NLRP3 interacts through its NACHT domains with the purinergic receptor P2Y2. Mainly detected on macrophages, this interaction is tightly regulated during cellular activation and enhanced during SIV or HIV-1 infections. They found that P2Y2-dependent migration of macrophages is repressed during NLRP3 inflammasome activation and that macrophage polarization, cytokine secretion and pyroptosis that involved NLRP3 activation are under the control of P2Y2-mediated (AMPK/c-CBL-dependent) autophagy. Thus, the results reveal that the interaction between NLRP3 and P2Y2 is essential for harnessing innate immunity and provide new therapeutic opportunities for preventing and treating systemic diseases including autoimmune disorders, inflammatory diseases, infections, and cancer.

Accordingly, one aspect of the present invention relates to a method of reducing macrophage M1 polarization in a subject in need thereof comprising administering to the subject a therapeutically effective amount of a P2Y2 receptor agonist.

As used herein the expression "reducing macrophage M1 polarization" means that the P2Y2 agonist of the present invention causes a decrease in the M1 macrophage activation pool and/or increase in M2 macrophages pool, preferably a decrease in the M1 macrophage activation pool and an increase in the pool of M2 macrophages. Thus, the M1/M2 ratio decreases. This can be indicated, as disclosed herein, by changes in the levels of factors that are associated with M1 and M2 macrophages. Accordingly, the method of the present invention inhibits macrophage IL-6, IL-23, and IL-1beta production by macrophages.

One aspect of the present invention relates to a method of reducing the secretion of inflammatory cytokines (e.g. IL-6, IL-23, and IL-1beta) by macrophages in a subject in need thereof comprising administering to the subject a therapeutically effective amount of a P2Y2 receptor agonist. In particular, the method is particularly suitable for reducing the secretion of IL-1beta.

One aspect the present invention relates to a method of increasing M2 macrophages pool in a subject suffering from conditions associated with undesirable M1 polarization comprising administering to the subject a therapeutically effective amount of a P2Y2 receptor agonist.

One aspect of the present invention relates to a method for driving macrophages towards a M2-type (macrophage M2 polarization) immune response and/or away from a M1-type (macrophage M1 polarization) immune response in patients comprises administering to the subject an effective amount of a P2Y2 receptor agonist.

One aspect of the present invention relates to a method of treating an inflammatory disease in a subject in need thereof comprising administering to the subject a therapeutically effective amount of a P2Y2 receptor agonist.

As used herein, the term "inflammatory disease" as used herein refers to acute or chronic localized or systemic responses to harmful stimuli, such as pathogens, damaged cells, physical injury or irritants, that are mediated in part by the activity of cytokines, chemokines, or inflammatory cells (e.g. macrophages) and is characterized in most instances by pain, redness, swelling, and impairment of tissue function. The inflammatory disease may be selected from the group consisting of: sepsis, septicemia, pneumonia, septic shock, systemic inflammatory response syndrome (SIRS), Acute Respiratory Distress Syndrome (ARDS), acute lung injury, aspiration pneumonitis, infection, pancreatitis, bacteremia, peritonitis, abdominal abscess, inflammation due to trauma, inflammation due to surgery, chronic inflammatory disease, ischemia, ischemia-reperfusion injury of an organ or tissue, tissue damage due to disease, tissue damage due to chemotherapy or radiotherapy, and reactions to ingested, inhaled, infused, injected, or delivered substances, glomerulonephritis, bowel infection, opportunistic infections, and for subjects undergoing major surgery or dialysis, subjects who are immunocompromised, subjects on immunosuppressive agents, subjects with HIV/AIDS, subjects with suspected endocarditis, subjects with fever, subjects with fever of unknown origin, subjects with cystic fibrosis, subjects with diabetes mellitus, subjects with chronic renal failure, subjects with bronchiectasis, subjects with chronic obstructive lung disease, chronic bronchitis, emphysema, or asthma, subjects with febrile neutropenia, subjects with meningitis, subjects with septic arthritis, subjects with urinary tract infection, subjects with necrotizing fasciitis, subjects with other suspected Group A streptococcus infection, subjects who have had a splenectomy, subjects with recurrent or suspected enterococcus infection, other medical and surgical conditions associated with increased risk of infection, Gram positive sepsis, Gram negative sepsis, culture negative sepsis, fungal sepsis, meningococcemia, post-pump syndrome, cardiac stun syndrome, stroke, congestive heart failure, hepatitis, epiglotittis, E. coli 0157:H7, malaria, gas gangrene, toxic shock syndrome, pre-eclampsia, eclampsia, HELP syndrome, mycobacterial tuberculosis, Pneumocystic carinii, pneumonia, Leishmaniasis, hemolytic uremic syndrome/thrombotic thrombocytopenic purpura, Dengue hemorrhagic fever, pelvic inflammatory disease, Legionella, Lyme disease, Influenza A, Epstein-Barr virus, encephalitis, inflammatory diseases and autoimmunity including Rheumatoid arthritis, osteoarthritis, progressive systemic sclerosis, systemic lupus erythematosus, inflammatory bowel disease, idiopathic pulmonary fibrosis, sarcoidosis, hypersensitivity pneumonitis, systemic vasculitis, Wegener's granulomatosis, transplants including heart, liver, lung kidney bone marrow, graft-versus-host disease, transplant rejection, sickle cell anemia, nephrotic syndrome, toxicity of agents such as OKT3, cytokine therapy, cryoporin associated periodic syndromes and cirrhosis.

One aspect of the present invention relates to a method of treating an auto-immune disease in a subject in need thereof comprising administering to the subject a therapeutically effective amount of a P2Y2 receptor agonist.

As used herein, an "autoimmune disease" is a disease or disorder arising from and directed at an individual's own tissues. Examples of autoimmune diseases include, but are not limited to Addison's Disease, Allergy, Alopecia Areata, Alzheimer's disease, Antineutrophil cytoplasmic antibodies (ANCA)-associated vasculitis, Ankylosing Spondylitis, Antiphospholipid Syndrome (Hughes Syndrome), arthritis, Asthma, Atherosclerosis, Atherosclerotic plaque, autoimmune disease (e.g., lupus, RA, MS, Graves' disease, etc.), Autoimmune Hemolytic Anemia, Autoimmune Hepatitis, Autoimmune inner ear disease, Autoimmune Lymphoproliferative syndrome, Autoimmune Myocarditis, Autoimmune Oophoritis, Autoimmune Orchitis, Azoospermia, Behcet's Disease, Berger's Disease, Bullous Pemphigoid, Cardiomyopathy, Cardiovascular disease, Celiac Sprue/Coeliac disease, Chronic Fatigue Immune Dysfunction Syndrome (CFIDS), Chronic idiopathic polyneuritis, Chronic Inflammatory Demyelinating, Polyradicalneuropathy (CIPD), Chronic relapsing polyneuropathy (Guillain-Barre syndrome), Churg-Strauss Syndrome (CSS), Cicatricial Pemphigoid, Cold Agglutinin Disease (CAD), chronic obstructive pulmonary disease (COPD), CREST syndrome, Crohn's disease, Dermatitis, Herpetiformus, Dermatomyositis, diabetes, Discoid Lupus, Eczema, Epidermolysis bullosa acquisita, Essential Mixed Cryoglobulinemia, Evan's Syndrome, Exopthalmos, Fibromyalgia, Goodpasture's Syndrome, Hashimoto's Thyroiditis, Idiopathic Pulmonary Fibrosis, Idiopathic Thrombocytopenia Purpura (ITP), IgA Nephropathy, immunoproliferative disease or disorder (e.g., psoriasis), Inflammatory bowel disease (IBD), including Crohn's disease and ulcerative colitis, Insulin Dependent Diabetes Mellitus (IDDM), Interstitial lung disease, juvenile diabetes, Juvenile Arthritis, juvenile idiopathic arthritis (JIA), Kawasaki's Disease, Lambert-Eaton Myasthenic Syndrome, Lichen Planus, lupus, Lupus Nephritis, Lymphoscytic Lypophisitis, Ménière's Disease, Miller Fish Syndrome/acute disseminated encephalomyeloradiculopathy, Mixed Connective Tissue Disease, Multiple Sclerosis (MS), muscular rheumatism, Myalgic encephalomyelitis (ME), Myasthenia Gravis, Ocular Inflammation, Pemphigus Foliaceus, Pemphigus Vulgaris, Pernicious Anaemia, Polyarteritis Nodosa, Polychondritis, Polyglandular Syndromes (Whitaker's syndrome), Polymyalgia Rheumatica, Polymyositis, Primary Agammaglobulinemia, Primary Biliary Cirrhosis/Autoimmune cholangiopathy, Psoriasis, Psoriatic arthritis, Raynaud's Phenomenon, Reiter's Syndrome/Reactive arthritis, Restenosis, Rheumatic Fever, rheumatic disease, Rheumatoid Arthritis, Sarcoidosis, Schmidt's syndrome, Scleroderma, Sjörgen's Syndrome, Stiff-Man Syndrome, Systemic Lupus Erythematosus (SLE), systemic scleroderma, Takayasu Arteritis, Temporal Arteritis/Giant Cell Arteritis, Thyroiditis, Type 1 diabetes, Type 2 diabetes, Ulcerative colitis, Uveitis, Vasculitis, Vitiligo, and Wegener's Granulomatosis.

One aspect of the present invention relates to a method for promoting secretion of inflammatory cytokines (e.g. IL-1beta, TNF, IL-12, and IL-23) by macrophage in a subject in need thereof comprising administering to the subject a therapeutically effective amount of an inhibitor of P2Y2 receptor activity or expression. In particular, the method is particularly suitable for promoting TH1 and TH17 cell inflammatory responses that participate to the clearance of invading microorganisms.

One aspect of the present invention relates to a method of treating an infectious disease in a subject in need thereof comprising administering to the subject a therapeutically effective amount of an inhibitor of P2Y2 receptor activity or expression.

In some embodiments, the method of the present invention is particularly suitable for the treatment of viral infections. As used herein, the term "viral infection" refers to any stage of a viral infection, including incubation phase, latent or dormant phase, acute phase, and development and maintenance of immunity towards a virus. Example of virus infections include infections mediated by Retroviridae (i.e. Lentivirinae), like HIV (human immunodeficiency virus); Flaviviridae, which comprises (i) the Flaviviruses like Yellow fever virus (YFV) and Dengue virus, the Hepaciviruses like HCV (hepatitis C virus) and (iii) the Pestiviruses like Bovine viral diarrhea virus (BVDV); Herpesviridae, like Herpes simplex virus type 1 (HSV-1) or type 2 (HSV-2), Varicella-zoster virus (VZV), Cytomegalovirus (CMV) or Human Herpes virus type 6 (HHV-6); Poxyiridae, like Vaccinia; Hepadnaviridae, like HBV (hepatitis B virus); Coronaviridae, like SARS-CoV; Orthomyxoviridae, like influenza virus A, B and C; Togaviridae; Arenaviridae, like Arenavirus; Bunyaviridae, like Punta Toro; Paramyxoviridae, like Respiratory syncytial virus (RSV) or Parainfluenza-3 virus; and Rhabdoviridae.

In some embodiments, treatment of HIV-1 infection is excluded from the scope of the present invention.

In some embodiments, the method of the present invention is particularly suitable for the treatment of bacterial infections. Examples of bacterial organisms against which the method of the present invention is effective include gram positive bacteria, gram negative bacteria, and acid fast bacteria, and particularly, *Staphylococcus aureus, Streptococcus pyogenes, Streptococcus pneumoniae, Mycobacterium* and *Escherichia coli.* The methods and compositions of the invention are effective against infection by all bacterial organisms, including members of the following genera: *Aerococcus, Listeria, Streptomyces, Chlamydia, Lactobacillus, Eubacterium, Arachnid, Mycobacterium, Peptostreptococcus, Staphylococcus, Corynebacterium, Erysipelothrix, Dermatophilus, Rhodococcus, Pseudomonas, Streptococcus, Bacillus, Peptococcus, Pneumococcus, Micrococcus, Neisseria, Klebsiella, Kurthia, Nocardia, Serratia, Rothia, Escherichia, Propionibacterium, Actinomyces, Helicobacter, Enterococcus, Shigella, Vibrio, Clostridium, Salmonella, Yersinia, and Haemophilus.*

In some embodiments, the method of the present invention is particularly suitable for the treatment of fungal infection. For example, a range of fungi or moulds, called dermnatophytes, cause fungal infections of the skin. These fungi are parasites on the skin and cause different symptoms in different parts of the body. They are very infectious and are passed from person to person. Although typically these infections are topical, in certain patients (e.g., immunosuppressed patients) they may occur systemically or in internally. Fungal infections that may be treated with the compositions of the present invention include dermatophytosis (*Trichophyton, Epidermophyton, and Microsporum*), candidiasis (*Candida albicans* and other *Candida* species), tinea versicolor (*Pityrosporum orbiculare*), tinea pedea (*Trichophyton mentagrophytes, Trichophyton rubrum, and Epidermophytonfloccosum*), tinea capitis and ringworm (*Trichophyton tonsurans*). In some embodiments, the method of the present invention is particularly suitable for the treatment of yeast infection. For example, vaginal yeast infections are generally caused by *Candida albicans,* which, along with a few types of bacteria, are normally present in relatively small numbers in the vaginal area. Sometimes the yeast multiply rapidly and take over, causing *candidiasis* or *monilia.* This is often due to a change in the vaginal environment, injury, sexual transmission, HIV infection, etc. Common environmental disruptions that favor yeast include increased pH, increased heat and moisture, allergic reactions, elevated sugar levels, hormonal fluxes, and reductions in the populations of bacteria that are normally present.

One aspect of the present invention relates to a method of reducing macrophage M2 polarization in a subject in need thereof comprising administering to the subject a therapeutically effective amount of an inhibitor of P2Y2 receptor activity or expression.

As used herein the expression "reducing macrophage M2 polarization" means that the P2Y2 antagonist of the present invention causes a decrease in the M2 macrophage activation pool and/or increase in M1 macrophages pool, preferably a decrease in the M2 macrophage activation pool and an increase in M1 macrophages pool. Thus, the M1/M2 ratio increases. This can be indicated, as disclosed herein, by changes in the levels of factors that are associated with M1 and M2 macrophages. Accordingly, the method of the present invention inhibits macrophage MCP-1, MMP-9 and IL-6 production by macrophages. In particular, the method of the present invention down regulates surface FcγRI (CD64) and FcγRIII (CD16) expression on macrophages. In particular, the method of the invention promotes IL-1b production by macrophages.

One aspect the present invention relates to a method of increasing M1 macrophages pool in a subject suffering from conditions associated with undesirable M2 polarization comprising administering to the subject a therapeutically effective amount of an inhibitor of P2Y2 receptor activity or expression of the present invention.

One aspect of the present invention relates to a method for driving macrophages towards a M1-type (macrophage M1 polarization) immune response and/or away from a M2-type (macrophage M2 polarization) immune response in patients comprises administering to the subject an effective amount of an inhibitor of P2Y2 receptor activity or expression.

One aspect of the present invention relates to a method of reducing macrophage pro-tumoral functions (i.e. tumorigenicity) and/or increasing macrophage tumor suppression activity in a patient, especially in patient suffering from conditions associated with undesirable M2 polarization comprising administering to the subject a therapeutically effective amount of an inhibitor of P2Y2 receptor activity or expression of the present invention. In some embodiments, the method of the present invention reduces tumor-associated macrophages (TAM) recruitment into tumor and/or at least one macrophage pro-tumoral functions. In some embodiments, the method of the present invention represses at least one macrophage pro-tumoral functions selected in the group consisting of tumor invasion, metastasis, tumor cell proliferation, tumor growth, tumor survival, neo-angiogenesis, suppression of innate or adaptive immunity and extracellular matrix remodeling; and tumor angiogenesis.

According to the present invention "conditions associated with undesirable M2 macrophage polarization" designate cancer, especially metastatic cancer, progressive fibrotic diseases such as for example idiopathic pulmonary fibrosis (IPF), hepatic fibrosis systemic sclerosis, allergy and asthma, atherosclerosis and Alzheimer's disease. In particularly, the method of the present invention is particularly suitable for the treatment of cancer. As used herein, the term "cancer" has its general meaning in the art and includes, but is not limited to, solid tumors and blood-borne tumors. The term cancer includes diseases of the skin, tissues, organs, bone, cartilage, blood and vessels. The term "cancer" further encompasses both primary and metastatic cancers. Examples of cancers that may be treated by methods and compositions of the invention include, but are not limited to, cancer cells from the bladder, blood, bone, bone marrow, brain, breast, colon, esophagus, gastrointestinal tract, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous; adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; Paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; Sertoli cell carcinoma; Leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malignant melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia. In some embodiments, the method of the present invention is particularly suitable for the treatment of metastatic cancer to bone, wherein the metastatic cancer is breast, lung, renal, multiple myeloma, thyroid, prostate, adenocarcinoma, blood cell malignancies, including leukemia and lymphoma; head and neck cancers; gastrointestinal cancers, including esophageal cancer, stomach cancer, colon cancer, intestinal cancer, colorectal cancer, rectal cancer, pancreatic cancer, liver cancer, cancer of the bile duct or gall bladder; malignancies of the female genital tract, including ovarian carcinoma, uterine endometrial cancers, vaginal cancer, and cervical cancer; bladder cancer; brain cancer, including neuroblastoma; sarcoma, osteosarcoma; and skin cancer, including malignant melanoma or squamous cell cancer.

As used herein, the term "P2Y2 receptor" has its general meaning in the art and refers to the P2Y purinoreceptor 2 which belongs to the family of G-protein coupled receptors (also abbreviated as P2Y2 receptor). Said receptor is encoded by the P2RY2 gene in humans as a G-protein-coupled receptor with the seven transmembrane-spanning domains. There are three human transcript variants which encode for the same 377 amino acid protein sequence.

As used herein, the term P2Y2 receptor agonist refers to any compound that enhances the biological activity of the P2Y2 receptor.

P2Y2 receptor agonists are well known in the art and include those described in U.S. Pat. No. 5,789,391, U.S. Pat. No. 5,837,861, and US 2002/0082417. Suitable P2Y2 agonists typically include INS-37217, uridine 5' triphosphate, diquafosol tetrasodium, and the like. INS37217 [P(1)-(uridine 5')-P(4)-(2'-deoxycytidine 5')tetraphosphate, tetrasodium salt] is a deoxycytidine-uridine dinucleotide with agonist activity at the P2Y2 receptor. In some embodiments, P2Y2 receptor agonists are selected from the compounds described in WO 2008060632 represented by one of the following: 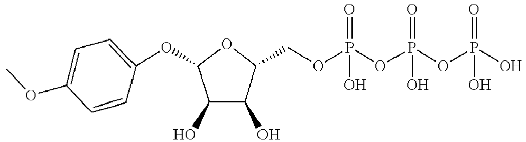 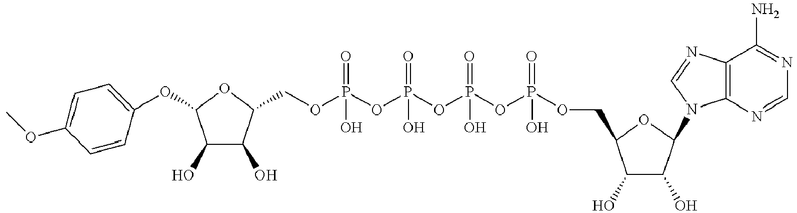 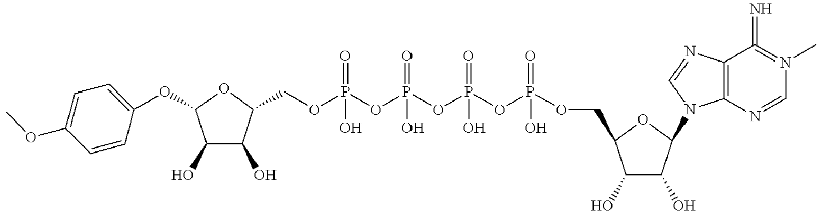 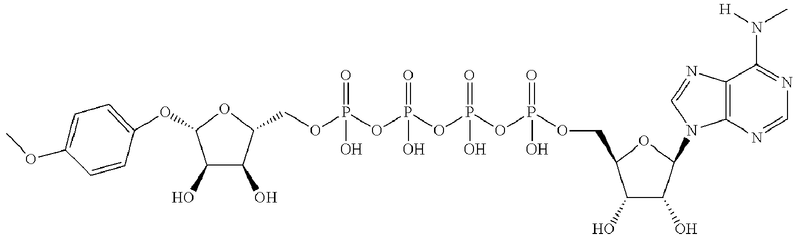 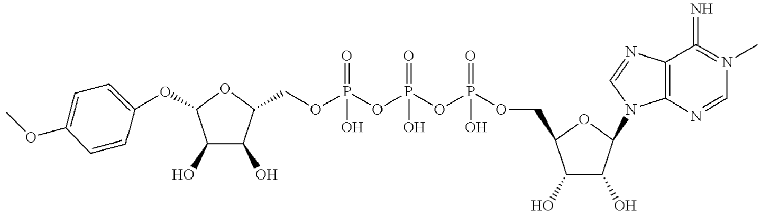 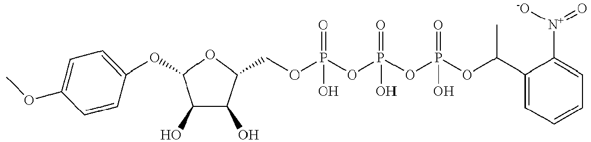 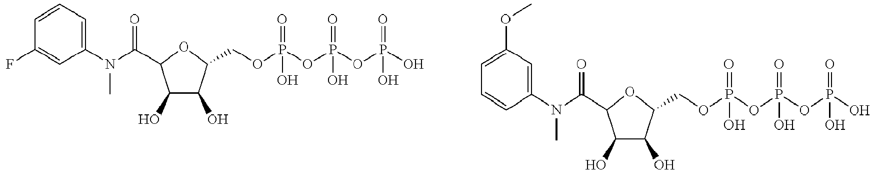 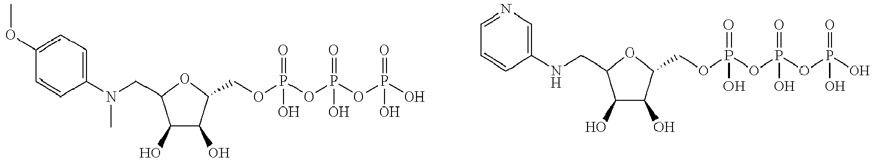 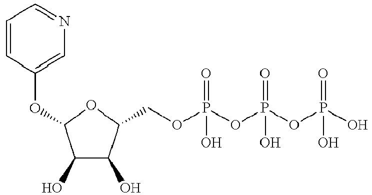 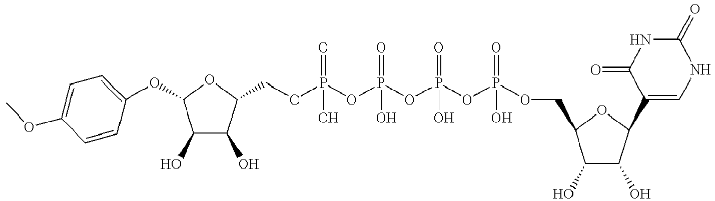 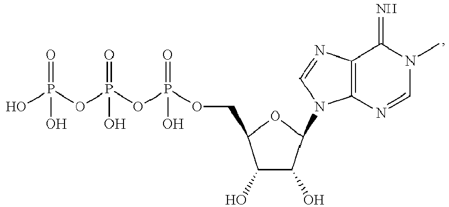 or a pharmaceutically acceptable salt thereof.

In some embodiments, the P2Y2 receptor agonist is an antibody directed against P2Y2 receptor.

In another embodiment P2Y2 receptor agonist of the invention is an aptamer.

As used herein the term "inhibitor of P2Y2 receptor activity or expression" refers to a compound that reduces or abolishes the biological function or activity of the P2Y2 receptor. An inhibitor may perform any one or more of the following effects in order to reduce or abolish the biological function or activity of P2Y2: (i) the transcription of the gene encoding P2Y2 receptor is lowered, i.e. the level of mRNA is lowered, (ii) the translation of the mRNA encoding P2Y2 receptor is lowered, (iii) P2Y2 receptor performs its biochemical function with lowered efficiency in the presence of the inhibitor, and (iv) the P2Y2 receptor performs its cellular function with lowered efficiency in the presence of the inhibitor. For example, such an inhibitor of P2Y2 receptor activity can act by occupying the binding site or a portion thereof of the P2Y2 receptor, thereby making the receptor inaccessible to its natural ligand (e.g. ATP) so that its normal biological activity is prevented or reduced. The antagonistic activity of compounds towards the P2Y2 receptors may be determined using various methods well known in the art. For example, the agents may be tested for their capacity to block the interaction of P2Y2 receptor with a natural ligand of P2Y2 receptor (e.g. ATP). For example, the assay is performed with P2Y2 receptor expressed on the surface of cells. A typical assay for determining the antagonistic activities of a compound on P2Y2 receptor is described in P. Hillmann, G.-Y. Ko, A. Spinrath, A. Raulf, I. von Kügelgen, S.C. Wolff, R.A. Nicholas, E. Kostenis, H.-D. Höltje and C.E. Müller, J. Med. Chem. 52 (2009), p. 27620. Briefly, the potency of the test compounds to inhibit UTP-induced intracellular calcium release in NG108-15 cells (mouse neuroblastoma × rat glioma hybrid cell line) may be determined by a fluorescence method using the calcium-chelating fluorophor Oregon Green®.

Exemplary small organic molecules that are inhibitor of P2Y2 receptor activity include but are not limited to uracil nucleotide analogs as described in Sauer R et al. (Sauer R, El-Tayeb A, Kaulich M, Müller CE. Synthesis of uracil nucleotide analogs with a modified, acyclic ribose moiety as P2Y(2) receptor antagonists Bioorg Med Chem. 2009 Jul 15;17(14):5071-9. Epub 2009 May 30) or 4-Phenylamino-substituted 1-amino-2-sulfoanthraquinones as described in Weyler S. et al. (Weyler S, Baqi Y, Hillmann P, Kaulich M, Hunder AM, Müller IA, Müller CE. Combinatorial synthesis of anilinoanthraquinone derivatives and evaluation as non-nucleotide-derived Inhibitor of P2Y2 receptor activitys. Bioorg Med Chem Lett. 2008 Jan 1;18(1):223-7. Epub 2007 Oct 30.) that are hereby incorporated by reference into the present disclosure. Typical Inhibitor of P2Y2 receptor activitys are diphosphoric 5-(2,4-dioxo-3,4-dihydropyrimidin-1(2H)-yl)pentylphosphonic anhydride, 4-phenyl-amino-4-(2-methoxyphenyl)-2-sulfoanthraquinone (PSB-716).

In some embodiments, the inhibitor of P2Y2 receptor activity consists in an antibody (the term including antibody fragment). In particular, the inhibitor of P2Y2 receptor activity may consist in an antibody directed against the P2Y2 receptor in such a way that said antibody impairs the activation of said receptor.

Antibodies can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred. Monoclonal antibodies can be prepared and isolated using any technique that provides for the production of antibody molecules by continuous cell lines in culture. Techniques for production and isolation include but are not limited to the hybridoma technique; the human B-cell hybridoma technique; and the EBV-hybridoma technique. Alternatively, techniques described for the production of single chain antibodies (see, e.g., U.S. Pat. No. 4,946,778) can be adapted to produce anti-P2Y2 receptor, single chain antibodies. The inhibitor of P2Y2 receptor activity of the invention also include anti-P2Y2 receptor antibody fragments including but not limited to F(ab')₂ fragments, which can be generated by pepsin digestion of an intact antibody molecule, and Fab fragments, which can be generated by reducing the disulfide bridges of the F(ab')₂ fragments. Alternatively, Fab and/or scFv expression libraries can be constructed to allow rapid identification of fragments having the desired specificity to the receptor or channel. Humanized antibodies and antibody fragments therefrom can also be prepared according to known techniques. "Humanized antibodies" are forms of non-human (e.g., rodent) chimeric antibodies that contain minimal sequence derived from non-human immunoglobulin. For the most part, humanized antibodies are human immunoglobulins (recipient antibody) in which residues from a hypervariable region (CDRs) of the recipient are replaced by residues from a hypervariable region of a non-human species (donor antibody) such as mouse, rat, rabbit or nonhuman primate having the desired specificity, affinity and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. Methods for making humanized antibodies are described, for example, by Winter (U.S. Pat. No. 5,225,539) and Boss (Celltech, U.S. Pat. No. 4,816,397).

Then after raising antibodies as above described, the skilled man in the art can easily select those activating or blocking the P2Y2 receptor.

In another embodiment the inhibitor of P2Y2 receptor activity of the invention is an aptamer.

Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library. The random sequence library is obtainable by combinatorial chemical synthesis of DNA. In this library, each member is a linear oligomer, eventually chemically modified, of a unique sequence. Peptide aptamers consists of a conformationally constrained antibody variable region displayed by a platform protein, such as E. coli Thioredoxin A that are selected from combinatorial libraries by two hybrid methods.

Then after raising aptamers directed against the P2Y2 receptor as above described, the skilled man in the art can easily select those activating or blocking the P2Y2 receptor.

An "inhibitor of gene expression" refers to a natural or synthetic compound that has a biological effect to inhibit or significantly reduce the expression of a gene.

Inhibitors of gene expression for use in the present invention may be based on anti-sense oligonucleotide constructs. Anti-sense oligonucleotides, including anti-sense RNA molecules and anti-sense DNA molecules, would act to directly block the translation of the mRNA by binding thereto and thus preventing protein translation or increasing mRNA degradation, thus decreasing the level of the protein (e.g. P2Y2 receptor), and thus activity, in a cell. For example, antisense oligonucleotides of at least about 15 bases and complementary to unique regions of the mRNA transcript sequence encoding the targeted protein (e.g. P2Y2 receptor) can be synthesized, e.g., by conventional phosphodiester techniques and administered by e.g., intravenous injection or infusion. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732).

Small inhibitory RNAs (siRNAs) can also function as inhibitors of gene expression for use in the present invention. Gene expression can be reduced by contacting a subject or cell with a small double stranded RNA (dsRNA), or a vector or construct causing the production of a small double stranded RNA, such that gene expression is specifically inhibited (i.e. RNA interference or RNAi). Methods for selecting an appropriate dsRNA or dsRNA-encoding vector are well known in the art for genes whose sequence is known (e.g. see Tuschl, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836).

Ribozymes can also function as inhibitors of gene expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.

Both antisense oligonucleotides and ribozymes useful as inhibitors of gene expression can be prepared by known methods. These include techniques for chemical synthesis such as, e.g., by solid phase phosphoramadite chemical synthesis. Alternatively, anti-sense RNA molecules can be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.

Antisense oligonucleotides siRNAs and ribozymes of the invention may be delivered in vivo alone or in association with a vector. In its broadest sense, a "vector" is any vehicle capable of facilitating the transfer of the antisense oligonucleotide siRNA or ribozyme nucleic acid to the cells and preferably cells expressing the targeted proteins (e.g. P2Y2 receptor). Preferably, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the antisense oligonucleotide siRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, 1990 and in Murry, 1991).

Preferred viruses for certain applications are the adeno-viruses and adeno-associated viruses, which are double-stranded DNA viruses that have already been approved for human use in gene therapy. The adeno-associated virus can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g. Sambrook et al., 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUCl9, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

One further aspect of the present invention relates to a method of treating an infectious disease in a subject in need thereof comprising administering to the subject a therapeutically effective amount of a LRR polypeptide or a nucleic acid molecule encoding thereof.

In particular the LRR polypeptide of the present invention is particularly suitable for the treatment of HIV-1 infection.

As used herein, the term "NLRP3" has its general meaning in the art and refers to the NACHT, LRR and PYD domains-containing protein 3. An exemplary human amino acid sequence is represented by SEQ ID NO:1. As used herein, the LRR domain has its general meaning in the art and is typically represented by the amino acid sequence ranging from the amino acid residue at position 742 to the amino acid residue at position 991 in SEQ ID NO:1.

SEQ ID NO:1 (canonical sequence of NLRP3_Homo Sapiens):



























As used herein, the term "nucleic acid molecule" has its general meaning in the art and refers to a DNA or RNA molecule. However, the term captures sequences that include any of the known base analogues of DNA and RNA such as, but not limited to 4-acetylcytosine, 8-hydroxy-N6-methyladenosine, aziridinylcytosine, pseudoisocytosine, 5-(carboxyhydroxylmethyl) uracil, 5-fiuorouracil, 5-bromouracil, 5-carboxymethylaminomethyl-2-thiouracil, 5-carboxymethyl-aminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5- methylcytosine, N6-methyladenine, 7-methylguanine, 5-methylaminomethyluracil, 5-methoxyamino-methyl-2-thiouracil, beta-D-mannosylqueosine, 5'-methoxycarbonylmethyluracil, 5-methoxyuracil, 2-methylthio-N6-isopentenyladenine, uracil- 5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, -uracil-5-oxyacetic acid methylester, uracil-5-oxyacetic acid, pseudouracil, queosine, 2-thiocytosine, and 2,6-diaminopurine.

In some embodiments, the nucleic acid molecule of the present invention is included in a suitable vector, such as a plasmid, cosmid, episome, artificial chromosome, phage or a viral vector. So, a further object of the invention relates to a vector comprising a nucleic acid encoding for a mutated FX polypeptide of the invention. Typically, the vector is a viral vector which is an adeno-associated virus (AAV), a retrovirus, bovine papilloma virus, an adenovirus vector, a lentiviral vector, a vaccinia virus, a polyoma virus, or an infective virus. In some embodiments, the vector is an AAV vector. As used herein, the term "AAV vector" means a vector derived from an adeno- associated virus serotype, including without limitation, AAV1, AAV2, AAV3, AAV4, AAV5, AAV6, AAV7, AAV8, AAV9, and mutated forms thereof. AAV vectors can have one or more of the AAV wild-type genes deleted in whole or part, preferably the rep and/or cap genes, but retain functional flanking ITR sequences. Retroviruses may be chosen as gene delivery vectors due to their ability to integrate their genes into the host genome, transferring a large amount of foreign genetic material, infecting a broad spectrum of species and cell types and for being packaged in special cell- lines. In order to construct a retroviral vector, a nucleic acid encoding a gene of interest is inserted into the viral genome in the place of certain viral sequences to produce a virus that is replication-defective. In order to produce virions, a packaging cell line is constructed containing the gag, pol, and/or env genes but without the LTR and/or packaging components. When a recombinant plasmid containing a cDNA, together with the retroviral LTR and packaging sequences is introduced into this cell line (by calcium phosphate precipitation for example), the packaging sequence allows the RNA transcript of the recombinant plasmid to be packaged into viral particles, which are then secreted into the culture media. The media containing the recombinant retroviruses is then collected, optionally concentrated, and used for gene transfer. Retroviral vectors are able to infect a broad variety of cell types. Lentiviruses are complex retroviruses, which, in addition to the common retroviral genes gag, pol, and env, contain other genes with regulatory or structural function. The higher complexity enables the virus to modulate its life cycle, as in the course of latent infection. Some examples of lentivirus include the Human Immunodeficiency Viruses (HIV 1, HIV 2) and the Simian Immunodeficiency Virus (SIV). Lentiviral vectors have been generated by multiply attenuating the HIV virulence genes, for example, the genes env, vif, vpr, vpu and nef are deleted making the vector biologically safe. Lentiviral vectors are known in the art, see, e.g.. U.S. Pat. Nos. 6,013,516 and 5,994,136, both of which are incorporated herein by reference. In general, the vectors are plasmid-based or virus-based, and are configured to carry the essential sequences for incorporating foreign nucleic acid, for selection and for transfer of the nucleic acid into a host cell. The gag, pol and env genes of the vectors of interest also are known in the art. Thus, the relevant genes are cloned into the selected vector and then used to transform the target cell of interest. Recombinant lentivirus capable of infecting a non-dividing cell wherein a suitable host cell is transfected with two or more vectors carrying the packaging functions, namely gag, pol and env, as well as rev and tat is described in U.S. Pat. No. 5,994,136, incorporated herein by reference. This describes a first vector that can provide a nucleic acid encoding a viral gag and a pol gene and another vector that can provide a nucleic acid encoding a viral env to produce a packaging cell. Introducing a vector providing a heterologous gene into that packaging cell yields a producer cell which releases infectious viral particles carrying the foreign gene of interest. The env preferably is an amphotropic envelope protein which allows transduction of cells of human and other species. Typically, the nucleic acid molecule or the vector of the present invention include "control sequences'", which refers collectively to promoter sequences, polyadenylation signals, transcription termination sequences, upstream regulatory domains, origins of replication, internal ribosome entry sites ("IRES"), enhancers, and the like, which collectively provide for the replication, transcription and translation of a coding sequence in a recipient cell. Not all of these control sequences need always be present so long as the selected coding sequence is capable of being replicated, transcribed and translated in an appropriate host cell. Another nucleic acid sequence, is a "promoter" sequence, which is used herein in its ordinary sense to refer to a nucleotide region comprising a DNA regulatory sequence, wherein the regulatory sequence is derived from a gene which is capable of binding RNA polymerase and initiating transcription of a downstream (3'-direction) coding sequence. Transcription promoters can include "inducible promoters" (where expression of a polynucleotide sequence operably linked to the promoter is induced by an analyte, cofactor, regulatory protein, etc.), "repressible promoters" (where expression of a polynucleotide sequence operably linked to the promoter is induced by an analyte, cofactor, regulatory protein, etc.), and "constitutive promoters".

By a "therapeutically effective amount" is meant a sufficient amount of the active agent (e.g. P2Y2 receptor agonist or the inhibitor of P2Y2 receptor activity or expression) at a reasonable benefit/risk ratio applicable to the medical treatment. It will be understood that the total daily usage of the compounds and compositions of the present invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

The active agent of the present invention (e.g. P2Y2 receptor agonist or the inhibitor of P2Y2 receptor activity or expression) is typically combined with pharmaceutically acceptable excipients, and optionally sustained-release matrices, such as biodegradable polymers, to form pharmaceutical compositions. The term "Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. In the pharmaceutical compositions of the present invention, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The active ingredient can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The invention will be further illustrated by the following figures and examples. However, these examples and figures should not be interpreted in any way as limiting the scope of the present invention.

### FIGURES:

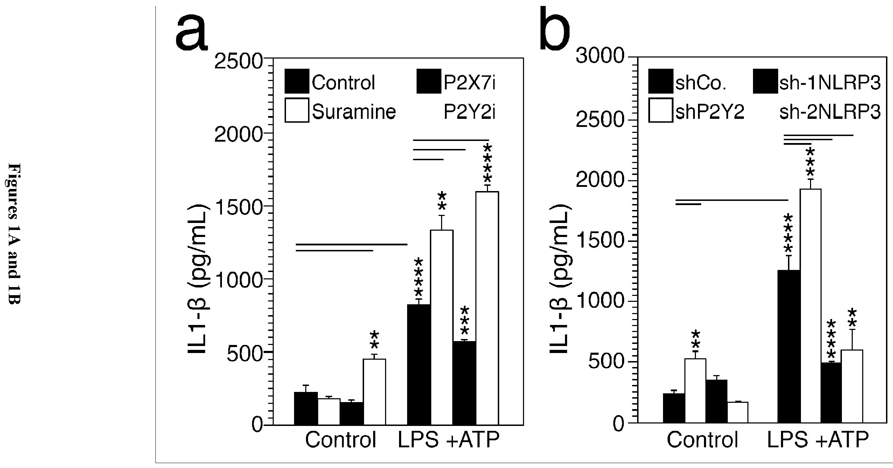

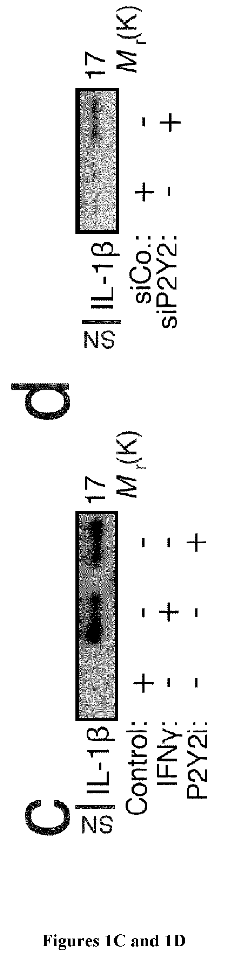

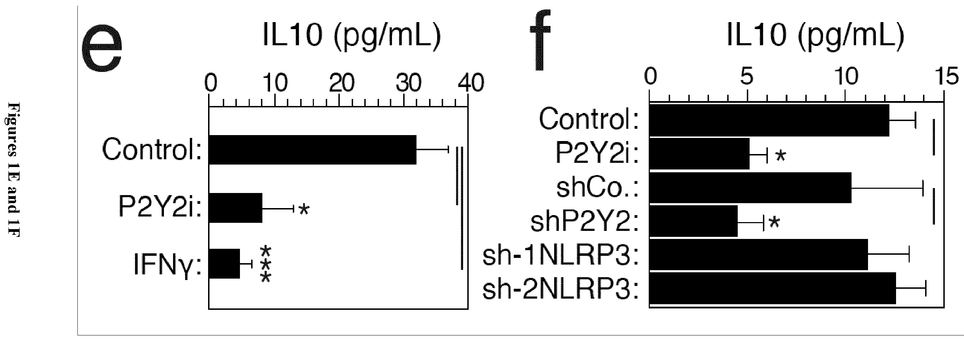

**Figure 1****: Antagonistic functions of P2Y2 and NLRP3 during macrophage activation.**
   a-f, LPS+ATP-stimulated PMA-primed THP-1 (a,b) or MDMs (c,d) treated with indicated drugs (a,c,e,f), IFNγ (c,e) or transfected with shRNA (b,f) or siRNA (d) were analysed for II-1β and IL-10 (a-f). Results shown in a,b,f and e were obtained respectively from 3 and 6 independent experiments. c and d show representative western blots of 6 and 3 independent experiments, respectively. Data are presented as means ± SEM in all panels except in c,d. Significances are * *P* ≤ 0.05, ** *P* ≤ 0.01, *** *P* ≤ 0.001 and **** *P* ≤ 0.0001 for all panels.

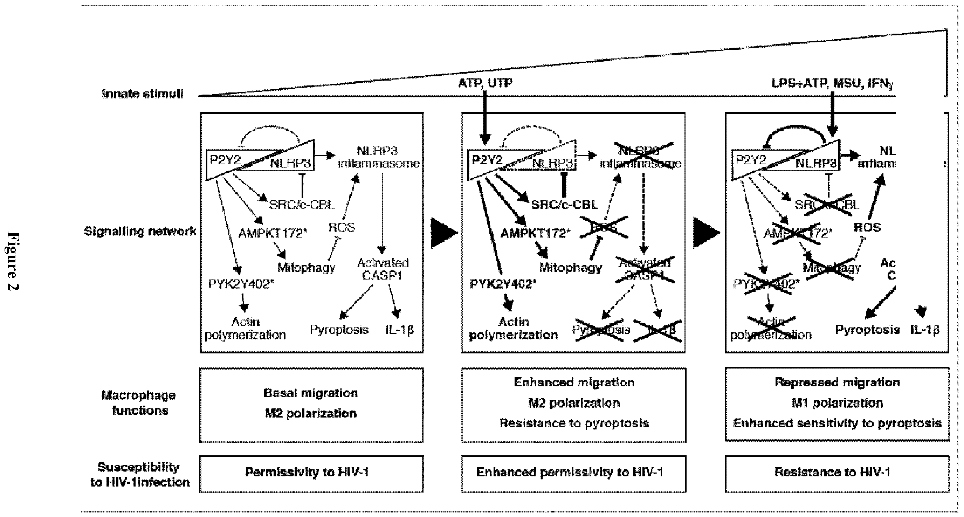

**Figure 2****: The proposed model in which NLRP3 levels control the functions and the susceptibility of macrophages to HIV-1 infection.**
   Upon macrophage differentiation, the NLRP3 protein and the purinergic receptor interact together and mediate a feedback loop that regulates migration, cytokine secretion and pyroptosis of M2 macrophages (A panel). Macrophages that are polarized to M2 phenotype reveal basal migration activity and cytokine secretion. These macrophages exhibit a normal susceptibility to HIV-1 infection. In presence of danger signals such as ATP or UTP (B panel), the membrane bound purinergic receptor P2Y2 of the macrophages is stimulated and induces the phosphorylations of SRC kinase on tyrosine 416 (SRCY416*), of the AMP kinase on threonine 172 (AMPKT172*) and of the PYK2 kinase on tyrosine 402 (PYK2Y402*). Once activated, the kinase SRC induces the proteosomal degradation of NLRP3 protein through c-CBL dependent process and the AMP kinase enhances autophagic flux and mitophagy thus allowing the elimination of damaged ROS-producing mitochondria. Altogether, these mechanisms impair NLRP3 inflammasome activation and enhance the PYK2Y402* that favour the F-actin cytoskeletal remodelling and macrophage migration. At this state, M2 macrophages exhibit resistance to pyroptosis and reveal an enhanced permissiveness to HIV-1 infection.

Conversely, stimulation of M2 macrophages with NLRP3 inflammasome inducers (such as LPS+ATP or MSU) or with IFNγ increases NLRP3 level, activates NLRP3/CASP1 inflammasome and induces pro-inflammatory II-1β secretion, allowing the polarization of M2 macrophages toward M1 phenotype (C panel). The increase of NLRP3 levels enhance macrophage sensitivity to pyroptosis and inhibits P2Y2-dependent signalling pathways that repress biological activity of NLRP3 including proteosomal c-CBL-dependent NLRP3 degradation and AMPK-dependent mitophagy. This mechanism controls also macrophage migration and resistance to HIV-1 infection by inhibiting PYK2Y402* phosphorylation and reducing F-actin polymerization, two cellular events that are involved in the entry of HIV-1 into macrophages.

### EXAMPLES:

### Material & Methods:

**Cells and culture conditions.** The monocyte cell line THP-1 and GFP-LC3⁺ THP-1 cells were maintained in RMPI-1640-Glutamax medium supplemented with 10% heat inactivated fetal bovine serum (FBS) and 100 UI/mL penicillin-streptomycin (Life technology). GFP-LC3⁺ THP-1 cells were obtained from J. Kehrl³⁵. HeLa cells stably transfected with the Env gene of HIV-1_{LAI/IIIB} (HeLa Env⁺), HeLa cells transfected with CD4 (HeLa CD4⁺CXCR4⁺) were selected in medium containing 500 µg/ml G418 and 293T cells were cultured in Dulbecco's modified Eagle's medium (DMEM)-Glutamax supplemented with 10% FBS and 100 UI/ml penicillin-streptomycin, in the absence or presence of the indicated concentrations of inhibitors. To generate Monocytes Derived Macrophages (MDMs) for HIV-1 infections, CD14⁺ monocytes were isolated from peripheral blood mononuclear cells (PBMCs) by positive selection using anti-CD14 beads (Miltenyi Biotec). Buffy coats from healthy donors were obtained from the French blood bank (Etablissement Français du Sang (EFS)). In accordance with French law, written informed consent to use the cells for clinical research was obtained from each donor. Purified monocytes were incubated in RMPI-1640-Glutamax medium supplemented with 100 UI/ml penicillin-streptomycin and with 10% FBS in the presence of 10 ng/ml recombinant human (rh) M-CSF (PeproTech). After 6 days of culture, adherent cells corresponding to the macrophages enriched fraction were harvested, washed and used for HIV-1 infection experiments. T cells were subsequently prepared from the monocyte depleted cell fraction of PBMCs. Peripheral blood lymphocytes (PBLs) were activated for 48 hours in fresh medium supplemented with 2.5 µg/ml PHA (Sigma-Aldrich) and 1 µg/ml rhIL-2 (PeproTech). PBLs were then washed and cultured in growth medium containing 1 µg/mL rhIL-2 for 24 hours before HIV-1 infections. For macrophage silencing and polarization assays, monocytes were separated from PBMCs by adherence to the plastic, detached and cultured for 6 days in hydrophobic Teflon dishes (Lumox Duthsher) in macrophage medium (RPMI 1640 supplemented with 200 mM L-glutamine, 100 U of penicillin, 100 µg streptomycin, 10 mM HEPES, 10 mM sodium pyruvate, 50 µM β-mercaptoethanol, 1% minimum essential medium vitamins, 1% non-essential amino acids (Life technology)) supplemented with 15% of heat inactivated human serum AB (Life technology). For experiments, MDMs were harvested and resuspended in macrophage medium containing 10% of FBS. MDMs obtained with this method are 91 to 96 % CD14⁺, they express: the differentiation markers (CD11b and CD71) and the M2 macrophage polarization markers (CD163 and CD206)³⁶.

**Plasmids and transfections.** NLRP3 coding sequence in the pUNO vector was purchased from InvivoGen. The Flag-tagged NLRP3 full length and its single domains PYD (1-93 aa), NACHT (220-546 aa) or LRR (742-991 aa) coding sequences were inserted in the 3xFlag-pcDNA3 and are a kind of gift from Gabriel Nunez³⁷. The sequence coding for P2Y2 (in the pEFGP-N1 vector) is a kind gift from Laura Erb³⁸. Transient transfections of HeLa CD4⁺CXCR4⁺ cells (2.4x10⁵) or 293T (3x10⁵) cells with mammalian expression vectors (1-5 µg) were performed using Fugene transfection reagent (Promega), following the manufacturer's instructions. Western blot, immunoprecipitation analyses and experiences of HIV-1 infection or cell fusion were performed 24 hours after transfection.

**Viral constructs and in vitro infection.** To produce stocks of wild type HIV-1_{NL4-3} or HIV-1_{AD8}, 293T cells (2x10⁶) were transfected with 20 µg of the corresponding proviral DNA (pNL4-3 or pAD8) and for Env-deleted VSV-G pseudotyped NL4-3 viruses (HIV-1_{NL4-3ΔEnv}), 293T cells (2x10⁶) cells were transfected with 4µg of VSV-G expression vector (pVSV-G) and 16 µg of HIV-1 proviral DNA (pNL4-3ΔEnv) by the calcium phosphate method. After 12 hours, the transfection mixture was replaced with 8 ml of fresh growth medium. Then, 24 hours later, the media containing the first batch of virus was harvested and 8 ml of fresh growth medium was added to the cells for additional 24 hours. Upon collection, all virus-containing media was low-speed centrifuged, filtered through a 0.45 µm pore size filter (Sartorius stedim), to remove cell debris, treated with Benzonase (Novagen®) and stored in 1 ml aliquots at -80°C³⁹⁻⁴¹. Stocks of wild type HIV-1_{NDK} and HIV-1_{BAL} were obtained as previously described⁴². Viral stocks were standardized by quantification of p24 antigen in cell culture supernatants with an enzyme-linked immunoabsorbent assay (ZeptoMetrix Corporation) and infection of TZM cells (bearing the β-galactosidase gene under the control of HIV-1 LTR) with serial dilutions of the stocks followed by cell fixation and X-Gal staining. After 3 days of infection with HIV-1_{NDK} (Multiplicity of infection (MOI) of 1), PHA/IL-2-stimulated peripheral blood lymphocytes were cocultured with uninfected lymphoblasts or alone for 48 hours and analysed by immunofluorescence for synapse formation. MDMs were infected with HIV-1_{BaL} during 3 or 6 days (with a MOI of 2) and analysed by Proximity Ligation Assay (PLA) (following manufacturer's instructions) or by ELISA p24 and intracellular p24 as previously described¹⁰. THP-1 cells were also infected with HIV-1_{NL4-3} (MOI of 1) or HIV-I_{NL4-3ΔEnv} (MOI of 1) during 6 hours and analysed for related signalling pathways by western blot. Target cell infectability was evaluated as previously described¹⁰ using the enhanced β-galactosidase assay kit (Roche).

**RNA interference.** Transient knockdowns of cell lines mediated by small interfering RNAs (siRNAs) were all purchased from Sigma. The siRNAs used in knockdown experiments had the following sequences: NLRP3, siRNA-1, 5'-GGAUCAAACUACUCUGUGA -3' (SEQ ID NO:2); siRNA-2, 5'-UGCAAGAUCUCUCAGCAAA -3' (SEQ ID NO:3) and control siRNA, 5' UUCAAUAAAUUCUUGAGGU -3' (SEQ ID NO:4). siRNAs transfection were performed with 20nM siRNA using Oligofectamine (Invitrogen) according to the manufacturer's instructions. Western blot analyses and experiences of HIV-1 infections or cell fusions were performed 48 hours after siRNA transfection. For short hairpin RNA (shRNA) lentiviral particles transduction, the pLKO.1 shRNA expression lentiviral vector coding for each targeted gene was purchased from Thermo Scientific. The shRNAs used in knockdown experiments had the following sequences: P2Y2, shRNA-1, 5' - ATGTTCCACCTGGCTGTGTCTGATGCACT - 3' (SEQ ID NO:5); NLRP3, shRNA-1, 5' - AAACCCAGGGCTGCCTTGGAAAAG - 3' (SEQ ID NO:6), shRNA-2, 5' - AAACCCAGGGCTGCCTTGGAAAAG - 3' (SEQ ID NO:7); ASC, shRNA-1, 5' - AAACCCAGGGCTGCCTTGGAAAAG - 3' (SEQ ID NO:8), shRNA-2, 5' - AAACCCAGGGCTGCCTTGGAAAAG - 3' (SEQ ID NO:9); CASP1, shRNA-1, 5' - AAACCCAGGGCTGCCTTGGAAAAG - 3' (SEQ ID NO:10), shRNA-2, 5' - AAACCCAGGGCTGCCTTGGAAAAG - 3' (SEQ ID NO:11) and pLKO.1 empty vector control (Open Biosystem). Lentiviral vector particles were generated by cotransfection of three plasmids coding for the gag-pol HIV-1 genes (pCMV GAG-POL HIV University of Michigan), for the vector genome carrying shRNA of interest (pLKO.1 shRNA) and for the plasmid coding for an envelope of VSVG (pMDG-VSV-G). Cotransfection was effected into 293T cells using Fugene transfection reagent (Promega) according to the manufacturer's protocol. Two days after transfection, supernatants were filtered using 0.45-µm cellulose acetate filters (Sartorius stedim), aliquoted and frozen at -80°C. For transduction, lentivirus productions were added to the monocytic THP-1 cell line (4x10⁶) or into CD4⁺CXR4⁺ cells (10⁶) and 24 hours after transduction the medium was replaced with fresh growth medium containing 1 µg/mL puromycin (Invivogen). For transfection of MDMs, siRNAs were purchased from Dharmacon. The siRNAs against P2Y2 is siGenome smart pool selected composed of a pool of four siRNAs having the following sequences: 1- 5' UGC CUA GGG CCA AGC GCAA 3' (SEQ ID NO: 12), 2-5' UAA CUG GAG CUC CGA UUU A 3' (SEQ ID NO:13), 3- 5' UCU CAG GAG UAG UCU CAU A 3' (SEQ ID NO:14), 4- 5' AGU CAU CGU UUG UGU GUA U 3' (SEQ ID NO:15). The control siRNAs are a pool of four on target plus non-targeting siRNAs. The transfection protocol was previously described³⁶. Briefly, MDMs were seeded (0.5x10⁶ MDMs/0.5 ml/ well of 12-well plate in macrophages medium + 10% FBS) and let to be attached at 37°C for 2 hours prior siRNAs transfection. The siRNAs transfections were performed with the INTERFERin (Polyplus Transfection). Different amounts of siRNAs were pre-diluted in 1 ml of Opti-MEM in which 20 µl of INTERFERin were added and the transfection mix was let to incubate at room temperature for 10 minutes. The transfection mix (250 µl) was added to 0.5x10⁶ MDMs at the final concentrations of 100 nM siRNAs for P2Y2. Equal amounts of the on target plus non-targeting siRNAs were added to the control MDMs. The MDMs were then incubated at 37°C for overnight. The medium was replaced with fresh macrophage medium supplemented with 10% FBS prior to the infections. At 48 hours post-siRNA transfection, cell lysates and supernatants were assayed for protein expression by western blot.

**Western blots and immunoprecipitations.** Cells were washed twice with PBS and lysed in appropriated buffer (250 mM NaCl, 0.1% NP-40, 5 mM EDTA, 10 mM Na3VO4, 10 mM NaF, 5 mM DTT, 3 mM Na4P2O7, 1 mM EGTA, 10 mM Glycerol phosphate, 10 mM Tris-Hcl (pH=7.5) and the protease and phosphatase inhibitors (Roche)). 10-40 µg of protein extracts were run on 4-12% or 10% SDS-PAGE and transferred at 4°C onto a nitrocellulose membrane (0.2 Micron). After incubation for 2 hours at room temperature with 5% nonfat milk or BSA (Bovine Serum Albumine) in Tris-buffered saline and 0.1% Tween 20 (TBS-Tween), membranes were incubated with primary antibody at 4°C overnight. Horseradish peroxidase-conjugated goat anti-mouse or anti-rabbit (SouthernBiotech) antibodies were then incubated for 1 hour 30 minutes and revealed with the enhanced ECL detection system (GE Healthcare). The primary antibodies against GFP (D5.1), PYK2 (5E2), PYK2Y402*, SRC (36D10), SRCY416*, AMPKα (F6), AMPKT172* (40H9) and LC3A/B were obtained from Cell Signaling. Primary antibodies against IL-1β, CASP1, ASC (TMS1), IRF5, PNX1, Actin and GAPDH were purchased from Abcam. The primary antibodies anti-CAp24 (42-50) and anti-gp120 (2G12) were from NIH (AIDS Research and Reference Reagent Program, Division of AIDS, NIAID). Antibody anti-NLRP3 was from Adipogen (Cryo-2), anti-P2Y2 was from Alomone, anti-ubiquitin (P4D1) was from Santa-Cruz, anti-TOM20 and anti-Flag were from Sigma. For immunoprecipitations, cell pellets were lysed 6 hours after infection or 24 hours after transfection in CHAPS buffer (50 mM Tris-HCl (pH=7.5), 0.50 M NaCl and 0.1% CHAPS) containing protease and phosphatase inhibitors. Anti-NLRP3 (Origene), anti-P2Y2 (Abcam) antibodies were incubated overnight at 4°C with the cell lysates. The complexes were precipitated by incubation with Protein G Sepharose™ 4 Fast Flow (GE Healthcare). For anti-Flag immunoprecipitations, anti-Flag® M2 affinity Gel (Sigma) were incubated for overnight at 4°C with the cell lysates. The precipitated complexes were then extensively washed, boiled and analysed by western blotting using mouse or rabbit TrueBlot® (eBioscience) antibodies and revealed with the enhanced ECL detection system (GE Healthcare). Western blots shown are representative of at least of three independent experiments.

**Immunofluorescence and flow cytometry.** For immunofluorescence, cells were fixed in 2% paraformaldehyde-PBS for 5 minutes, permeabilized in 0.3% Triton (Sigma) in PBS, and incubated with PBS-FBS 20% for 1 hour, as previously described³⁹. Mitochondrial fragmentation and actin polymerization were analysed respectively using Mitotracker® Green FM (20 nM, Invitrogen) and Alexa Fluor 488 Phalloidin. For immunohistochemistry, 4-µm sections were cut from the paraffin blocks of the paraformaldehyde fixed tissues from mice, *Macaca fascicularis* or humans. After paraffin removal, slides were subjected to antigen retrieval by microwave boiling in 1 mmol/l EDTA pH 9.0. Slides were incubated with anti-P2Y2 (Alomone), anti-NLRP3 (Abcam), anti-CD163 (BD laboratories), anti-gp120 (2G12), anti-CD40 (BD laboratories) or anti-CASP1 p10 (Santa Cruz) overnight after permeabilization in 0.3% Triton for 5 minutes and saturation in PBS-FBS 20% for 1 hour. Then, with IgG conjugated to Alexa Fluor 488, 546 or 647 fluorochromes at room temperature during 1 hour and 30 minutes (life technologies). Proximity Ligation Assay (DUOLINK®, Sigma) was performed according to the manufacturer's instructions. Cells were analysed by fluorescent confocal microscopy on Leica SPE (using a 63X objective). Z series of optical sections at 0.4-µm increments were acquired. For flow cytometry, THP-1 cells (10⁶ cells/ml) were harvested in RPMI complete medium, washed twice with PBS, saturated at 4°C for 20 min with PBS-FBS 10% and incubated with anti-CD4 (FITC), anti-CD 184 (CXCR4) (PE-Cy5), anti-CD 195 (CCR5) (PE-Cy7) antibodies. The indicated antibodies and isotype-matched antibodies used were obtained from BD Pharmingen. Phenotypic analyses on primary human MDMs infected by HIV-1_{BaL} were realized by flow cytometry using mAb anti-CD163 (FITC), anti-CD206 (APC) and anti-p24 (PE). Mice alveolar macrophages were dissociated from lung (using Lung Dissociation kit from Miltenyi Biotech) and analysed using anti-CDllb (APC-Cy7) (BD laboratories), anti-CDllc (PE-Cy7) (BioLegend), anti-CD40 (eFluo710) (BioLegend), anti-F4/80 (FITC) (ebioscience) and anti-GR-1 (PE) (ebioscience).

**M1 polarization assays.** M2 MDMs that express at 100% the differentiation marker CD11b and the scavenger receptor M2 marker CD163 were then seeded (0.5x10⁶/ml in macrophage medium + 10% FBS), let to be attached on the plates for 2 hours at 37 °C before treatments with interferon γ (IFNγ) (1 µg/0,5x10⁶ MDMs), kaempferol (100 µM) or DMSO for control cells. MDMs and supernatants were harvest for flow cytometry and confocal microscopy analysis at 24 hours post-kaempferol treatments and for cytokines IL-10 ELISA analyses at 96 hours post-kaempferol treatments. Expressions of CD163 and IRF5 were respectively determined by flow cytometry and western blot. Release of II-1β and IL-10 was determined as indicated above by ELISA.

**Quantification of LDH release and cytokine secretion by ELISA.** Human THP-1 cells were cultured in RPMI 1640 media, supplemented with 10% FBS. THP-1 cells were differentiated by treatment for 3 hours with 100 nM phorbol-12-myristate-13-acetate (PMA, Invivogen). After 2 days THP-1 cells were stimulated first 3 hours with ultrapure LPS from E.coli (10 ng/ml, LPS) and then stimulated for 6 hours with ATP (5 mM, Sigma). Supernatants were used for cytokine assays and cell lysates for western blot analysis. Kaempferol (100µM, Sigma), OxATP (100µM, Sigma), Suramine (100µM, Sigma), or Bafilomycine A1 (50µM, TOCRIS) were added during LPS+ATP stimulation, while, PP1 (20µM, Sigma), PP2 (20µM, Sigma), MnTBAP (10µM, Sigma) and NAC (100µM, Sigma) 18 hours before LPS+ATP stimulations. Commercially available ELISA kits for IL-1β (Ebioscience), IL-10 (BD) or LDH (Roche) were used according to the manufacturers' instructions.

**Macrophage migration assays.** The PMA-primed THP-1 cell migration assay was performed in a Boyden chamber system (Roche CIM16 plate XCELLigence DP) during LPS (10ng/mL) and ATP (5mM) stimulation.

**Quantification of ROS production.** Mitochondria-associated ROS levels were measured by staining cells with H2DCFDA at 5 µM for 1hour or with MitoSOX at 0.25 µM for 10 minutes at 37 °C. Cells were then washed twice with HBSS solution and suspended in cold HBSS solution containing 1% FBS for FACS analysis²⁵.

**Mice.** Two weeks old *P2y2*^{+/+} and *P2y2*^{*-*/*-*} transgenic mice were obtained from Dr. Isabelle Couillin⁴³ and sacrificed upon arrival following the Federation of European Laboratory Animal Science Association guidelines and in accordance with the Ethical Committee of the Gustave Roussy Cancer Campus (CEE A26) (Villejuif, France). After sacrifice, plasmatic serum was stored at -80°C and lung biopsies were either fixed or digested for analysis as previously described⁴⁴.

**Histological analyses.** Samples from lymph nodes, ileum and colon tissues were obtained from*Macaca fascicularis* that have been infected by intrarectal inoculation with a single dose of 50 50% animal infectious doses (AID₅₀) of SIV_{mac251}. Adult cynomolgus macaques (*Macaca fascicularis*) were imported from Mauritius and housed in the facilities of the "Commissariat a l'Energie Atomique et aux Energies Alternatives" (CEA, Fontenay-aux-Roses, France). Non-human primates (NHP, which includes M. *fascicularis*) were used at the CEA in accordance with French national regulation and under national veterinary inspectors (CEA Permit Number A 92-032-02). The CEA is in compliance with Standards for Human Care and Use of Laboratory of the Office for Laboratory Animal Welfare (OLAW, USA) under OLAW Assurance number #A5826-01. The use of NHP at CEA is also in accordance with recommendation of the European Directive (2010/63, recommendation N°9). Animals were housed in adjoining individual cages allowing social interactions, under controlled conditions of humidity, temperature and light (12-hour light/12-hour dark cycles). Water was available *ad libitum.* Animals were monitored and fed 1-2 times daily with commercial monkey chow and fruits by trained personnel. Macaques were provided with environmental enrichment including toys, novel foodstuffs and music under the supervision of the CEA Animal Welfare Body. The protocols employed were approved by the ethical committee of the CEA "Comité d'Ethique en Experimentation Animale" registered the French Research Ministry under number 44. The animals were used under the supervision of the veterinarians in charge of the animal facility. Experimental procedures were conducted after animal sedation with ketamine chlorydrate (Rhone-Merieux, Lyon, France, 10 mg/kg) as previously described⁴⁵. Tissues were collected during animal necropsy (for SIV_{mac251}-infected animals, on days 701 to 738 after SIV infection) after sedation of the animals (ketamine chlorhydrate 10 mg/kg) followed by euthanasia (injection of sodium pentobarbital 180 mg/kg). Human autopsies from axillary lymph nodes and frontal cortex were obtained in accordance with the Italian and EU legislations, after approval by the Institutional Review Board of the National Institute for Infectious Disease « Lazzaro Spalanzani ». Lung autopsies from *P2y2*^{+/+} and *P2y2*^{*-*/*-*} mice were obtained and fixed as described above. 4-µm sections were cut from the paraffin blocks of the paraformaldehyde fixed human, NHP or mice tissues, prepared and analysed as described in "Immunofluorescence and flow cytometry" section.

**Statistical analysis.** All values were expressed as the mean ± SEM of cell individual samples. Samples values were analysed using Student's t-test for two groups, ANOVA for multiple groups or Mann -Whitney test for human MDMs donors. (GraphPad Prism version 6.0b; GraphPad Software).

### Results

### Direct interaction between NLRP3 and P2Y2 is enhanced during SIV and HIV infections.

Based on our previous work that highlighted the purinergic receptor P2Y2 as the central purinergic receptor contributing to the early steps of HIV-1 infection¹⁰, we decided to explore the putative functional impact of P2Y2 interacting proteins. In this context, we first paid particular attention to the NACHT, LRR and PYD domains-containing protein 3 (NLRP3), which is a major component of innate immune responses¹⁴. Indeed, NLRP3 can form an oligomeric complex with two other proteins, ASC and caspase-1 (CASP1), thus constituting the inflammasome, a caspase-1 activating complex that is required for proteolytic maturation and subsequent secretion of interleukin-1β (IL-1 β) and other pro-inflammatory cytokines¹⁵. Importantly, we detected that NLRP3 and P2Y2 co-immunoprecipitated (24 hours after transfection of Flag-tagged full-length NLRP3 with GFP-tagged full-length P2Y2) through an interaction that involves the NACHT domain (but not PYD and LRR domains) of NLRP3. To determine whether this interaction might occur during the early steps of HIV-1 infection, we studied the subcellular localization of NLRP3 after co-culture of HIV-1 infected lymphoblasts with uninfected cells. NLRP3 polarized towards the contact sites between HIV-1 infected and uninfected host cells, where it colocalized with the HIV-1 glycoprotein gp120 and with the purinergic receptor P2Y2. Thus, NLRP3 accumulates at the virological synapse that is formed between HIV-1 infected and uninfected lymphoblasts. Immuno-reactive NLRP3 was detected at higher levels in lymph nodes and in the frontal cortex from untreated HIV-1 carriers, as compared with uninfected specimens. Similar results were obtained when comparing control tissues from non-human primates (NHP) *Macaca fascicularis* to lymph nodes or intestinal tissues from NHP infected with a pathogenic simian immunodeficiency virus (SIV) strain, SIV_{mac251}. Considering that NLRP3 expression was preferentially observed in CD163⁺ macrophages that highly expressed P2Y2, we determined the ability of NLRP3 to directly interact with P2Y2 by means of a proximity ligation assay (PLA). This assay revealed that the interaction between NLRP3 and P2Y2 augmented in the ileum and the colon from SIV_{mac251}-infected (as compared to uninfected tissues), in the frontal cortex from untreated HIV-1 carriers (as compared with uninfected donors), at the contact sites between HIV-1_{NDK} infected and uninfected lymphoblasts, as well as in cell-free infections of lymphoblasts or of macrophages. Accordingly, NLRP3 and P2Y2 co-immunoprecipitated 6 hours after infection of human monocytes THP-1 with HIV-1_{NL4}-3.

### NLRP3 and P2Y2 antagonist activities dictate functional proprieties of macrophages.

Considering that NLRP3 and P2Y2 have been separately involved in macrophage activation and differentiation¹⁶⁻¹⁸, we investigated the reciprocal impact of NLRP3 or P2Y2 inactivation on distinct macrophage functions. Pharmacological inhibition of P2Y2 with suramin (a non-specific P2X and P2Y antagonist) or with kaempferol (a specific P2Y2 inhibitor, P2Y2i) (Fig. 1a,c), or depletion of P2Y2 by means of specific small interfering RNA (Fig. 1b,d) increased II-1β secretion by PMA-treated THP-1 macrophages stimulated with lipopolysaccharide and ATP (LPS+ATP) or by M2 human monocyte derived macrophages (MDMs), while inactivation of another purinergic receptor P2X7 (with oxidized ATP, P2X7i), or that of NLRP3 inhibited IL-1β secretion (Fig. 1a,b). Conversely, the specific P2Y2 agonist, MRS2768 impaired II-1β secretion after stimulation with LPS+ATP. Overall, these results indicate that P2Y2 represses NLRP3 inflammasome dependent II-1β secretion. Since IL-1β secretion is a hallmark of M1-macrophage type, we next analysed the impact of P2Y2 inactivation on functional reprogramming of macrophages¹⁹. P2Y2 inhibition (by kaempferol (Fig. 1c,e) or P2Y2 depletion (Fig. 1d,f) favoured the M1 polarization of PMA-treated THP-1 macrophages (Fig. 1f) and of MDMs (Fig. 1e) as revealed by the induction of II-1β secretion (Fig. 1c,d), the reduced secretion of the M2 cytokine interleukin-10 (IL-10) (Fig. 1e,f), the diminished membrane expressions of the M2 markers such as CD163 and CD20 and the increased the expression of IFN regulatory factor 5 (IRF5)²⁰. Indeed, P2Y2 inactivation or depletion induced M1-macrophage polarization at the same extent with the classical interferon gamma (IFNγ) M1 stimulation (Fig. 1c,e). These results indicate that P2Y2 represses M1 polarization. Interestingly, the baseline plasma II-1β concentrations from *P2y2*^{*-*/*-*} mice were higher than those found in wild type controls, suggesting that P2Y2 acts to suppress the (NLRP3-dependent) production of IL-1β. *P2y2*^{*-*/*-*} mice also exhibited a significant reduction in the frequency of M1 broncho-alveolar (CD11b⁺GR1⁻ F4/80⁺CD11c^{high}CD40^{high}). The vast majority of residual *P2y2*^{*-*/*-*} broncho-alveolar CD40⁺ M1 macrophages exhibited nuclear DNA fragmentation (as revealed by the TUNEL assay) and contained cleaved caspase-1 , suggesting that such *P2y2*^{*-*/*-*} macrophages were eliminated through pyroptosis²¹. Accordingly, granulocyte-macrophage colony-stimulating-factor (GM-CSF)-treated CD14⁺ bone marrow-derived monocytes (BMDM) obtained from *P2y2*^{*-*/*-*} mice released lactate dehydrogenase (LDH) as a marker for cell death upon LPS+ATP and similarly PMA-differentiated THP-1 macrophages stimulated with LPS+ATP lost their viability upon treatment with P2Y2i or P2Y2 depletion, showing an increased mortality in comparison with the control. This cell death was inhibited when either NLRP3 or CASP1 was depleted, in the line with the interpretation that P2Y2 inactivation induces NLRP3/CASP1-dependent macrophage pyroptosis. The depletion of NLRP3 (or that of its interacting proteins, ASC or CASP1) increased the P2Y2-dependent migration of macrophages induced by LPS+ATP. Altogether these results revealed that NLRP3 and P2Y2 antagonize each other as they regulate macrophage survival and functions.

### Mutually inhibitory feedback loops control NLRP3- and P2Y2-dependent signalling pathways.

To further characterize the antagonism between NLRP3 and P2Y2, we analysed the impact of P2Y2 inhibition (with kaempferol or P2Y2 knockdown) on the generation of reactive oxygen species (ROS) that contribute to NLRP3 inflammasome activation after LPS+ATP stimulation^{22,23}. P2Y2 inactivation indeed increased the LPS+ATP-induced ROS production detectable with 2',7'-dichlorodihydrofluorescein diacetate (*H2DCFDA*) or MitoSOX, a fluorescent sensor that is particular sensitive to mitochondrial ROS. P2Y2 inactivation also caused fragmentation of the mitochondrial network of LPS+ATP-stimulated PMA-treated THP-1 macrophages, as detected by fluorescence microscopic detection of the mitochondrial markers TOM20 or Mitotracker Green. Upon P2Y2 inactivation, positive correlations between II-1β secretion (Fig. 1a,b), mitochondrial ROS production and damaged mitochondria were observed, in line with the interpretation that ROS produced by damaged mitochondria may favour II-1β secretion in the context of P2Y2 inhibition. Indeed, both the antioxidant N-Acetyl Cysteine (NAC) and the superoxide dismutase (SOD) mimetic (MnTBAP) blunted the exacerbated II-1β secretion of P2Y2-inhibited THP-1 macrophages responding to LPS+ATP stimulatio. Hence, the accumulation of mitochondrial ROS that is detected when P2Y2 is repressed, contributes to II-1β secretion.

Considering that damaged ROS-generating mitochondria can be removed by autophagy (mitophagy) to avoid cellular damages^{24,25}, we speculated that P2Y2 might suppress mitophagy. The autophagosome marker LC3⁺ accumulated within cytoplasmic puncta around mitochondria after stimulation of LPS+ATP²⁵, and this effect was attenuated upon P2Y2 inactivation, correlating with the accumulation of damaged mitochondria. P2Y2 inactivation or P2Y2 knockdown did not only inactivate canonical P2Y2-dependent signalling pathways including the phosphorylation of SRC on tyrosine 416 (SRCY416*) or that of PYK2 on tyrosine 402 (PYK2Y402*) but also alleviated the pro-autophagic phosphorylation of the AMP-activated protein kinase on threonine 172 (AMPKT172*). This result was obtained in both LPS+ATP-stimulated THP-1 macrophages and primary human MDMs²⁶. In addition, the stimulation of IL-1β maturation by P2Y2i could be avoided by addition of the autophagy inducer rapamycin, underscoring the likelihood that P2Y2-regulated autophagy controls NLRP3 inflammasome activation. In the context of LPS+ATP stimulation, NLRP3 depletion enhanced the phosphorylation events (SRCY416* and PYK2Y402*) that occur in the canonical P2Y2 signalling pathway, as it induced AMPK activation (AMPKT172*). Concomitantly, NLRP3 depletion increased F-actin cytoskeletal remodelling, a cellular process that depends on PYK2Y402* and contributes to macrophage migration²⁷. Altogether, these data highlight the complex antagonistic crosstalk between NLRP3 and P2Y2.

### The interaction between NLRP3 and P2Y2 is controlled by SRC activation.

Knockdown of P2Y2 caused enhanced expression of NLRP3, while depletion of NLRP3 upregulated P2Y2 expression. To define the molecular mechanisms accounting for this reciprocal regulation, 293T cells were transfected with full-length NLRP3 alone or in combination with GFP-tagged full-length P2Y2. Co-expression of GFP-P2Y2 increased the ubiquitinylation levels and reduced the expression of NLRP3 protein, while co-expression of NLRP3 failed to reduce the expression of GFP-P2Y2. The down regulation of NLRP3 by P2Y2 was partially inhibited by the ubiquitin isopeptidase inhibitor G5 and fully suppressed by the proteasome inhibitor MG132. The ubiquitinylation and subsequent degradation of NLRP3 can be initiated by ubiquitin ligases such as E3 Ubiquin ligase c-CBL^{28,29}, but the signalling pathways that ignite this process remain elusive. We found that one P2Y2-associated signalling molecule, the kinase SRC was associated with NLRP3 when NLRP3 was ubiquitinated and degraded, suggesting that SRC might control NLRP3 degradation. Indeed, pharmacological inhibition of SRC with PP1 or PP2 impaired NLRP3 degradation. Conversely, expressions in 293T cells of full-length SRC together with full-length NLRP3 and GFP-tagged full-length P2Y2 led to the NLRP3 degradation. Considering that the SRC kinase was recently identified as a candidate for proteasomal degradation through a c-CBL-dependent process³⁰, we next investigated the role of c-CBL in the P2Y2-induced degradation of NLRP3. Indeed, c-CBL depletion abolished the negative effect of GFP-P2Y2 on the overall abundance of the NLRP3 protein. Altogether, these results suggest that P2Y2 controls NLRP3 degradation through a pathway involving SRC and the c-CBL ubiquitin ligase.

### NLRP3 inflammasome acts as an inducible restriction complex for HIV-1 infection.

Intrigued by the aforementioned observations, we determined whether the physical interaction between NLRP3 and P2Y2 is modulated during the early steps of HIV-1 infection. Six hours post-infection of human monocytes THP-1 with HIV-1_{NL4-3}, a fraction of NLRP3 that immunoprecipitated with P2Y2 was ubiquitinated. The ubiquitin isopeptidase inhibitor G5 increased the ubiquitinylation/degradation of NLRP3 and enhanced the HIV-1 permissiveness of human monocytes THP-1, as indicated by the increased intracellular accumulation of HIV-1 encoded protein p24. Next, we determined the effects of NLRP3 on viral replication. The activation of NLRP3 inflammasome by LPS alone or monosodium urate crystals (MSU) reduced the p24 antigen release and the replication of R5-tropic HIV-1_{BaL} in human MDMs. Conversely, depletion of NLRP3, ASC or CASP1 from human MDMs or PMA-differentiated THP-1 macrophages increased their infectability by HIV-1. These results were obtained using distinct HIV-1 isolates, namely, R5-tropic HIV-1_{AD8} and X4-tropic HIV-1_{NL4-3}, by means of several distinct readouts to measure HIV-1 infection, namely, the release of the p24 antigen, the amount of infectious HIV-1 particles generated by cells and the intracellular accumulation of p24 in THP-1 cells. In contrast, the depletion of NLRP3, ASC or CASP1 from THP-1 cells failed to increase cellular infectability by HIV-1_{NL4-3} variant in which the endogenous Env gene had been replaced by that of vesicular stomatitis virus (VSV; HIV-I_{NL4-3ΔEnv}), allowing the viral entry through the endocytic pathway³¹. These results indicate that the NLRP3 inflammasome only represses receptor-mediated P2Y2-dependent, not endocytic entry of HIV-1. However, depletion and overexpression of NLRP3 enhanced and reduced, respectively, the HIV-1 Env-elicited fusion and infection process without modulating the expression of the HIV-1 receptor CD4 and its co-receptor CXCR4. Upon P2Y2 depletion, NLRP3 accumulated and correlated with restricted HIV-1 infection. Conversely, NRLP3-, ASC- or CASP1-depleted THP-1 cells infected with HIV-1_{NL4-3} overactivated the P2Y2-dependent signalling pathway (as revealed by the enhanced expression of P2Y2 and the increased phosphorylation levels of PYK2Y402* and AMPKT172*) as compared to THP-1 control cells. Overexpression of Flag-tagged LRR domain of NLRP3 (but not that of its NACHT and PYD domains) inhibited the P2Y2-dependent signalling pathway during the early steps of HIV-1 infection and reduced the infectability of HIV-1 target cells. Altogether our data underline that NLRP3 inflammasome acts as an inducible restriction complex for HIV-1 infection.

### Discussion:

Deciphering the complex network of innate signalling pathways is a crucial step towards understanding the functionality of immune cells (such as monocytes, macrophages and dendritic cells) that might constitute prime targets for prophylactic or therapeutic intervention on infectious diseases. Here, we identified that two major sensors of danger signals NLRP3 and P2Y2, physically interact and exhibit mutually inhibitory functions and reciprocally control their expression levels. Stimulation of myeloid cells with P2Y2 agonists favoured the ubiquitinylation and subsequent proteosomal degradation of NLRP3, thus impairing the NLRP3-dependent secretion of pro-inflammatory II-1β secretion. P2Y2 inhibition did not only repress macrophage migration (as previously described¹⁷), but also enhanced II-1β secretion (presumably through the accumulation of ROS generating mitochondria as a consequence of impaired mitophagy). Conversely, activation of NLRP3 inflammasome attenuated P2Y2-dependent signalling pathways and hence negatively affected phosphorylation events downstream of P2Y2. Moreover, NLRP3 depletion favoured macrophage migration through PYK2-dependent F-actin polarization. Thus, our data highlighted a new network of opposing signals that regulated the function of myeloid cells (Fig. 2).

Innate immune signals reportedly play contrasting roles during the acute and chronic phases of HIV-1 infection⁷⁻⁹. Our data support that innate signalling networks are rerouted by HIV-1 during the early steps of infection. The interaction between NLRP3 and P2Y2 rapidly increase after HIV-1 infection cumulating in the ubiquitinylation and subsequent degradation of NLRP3, thus favouring the P2Y2-dependent viral entry into target cells. Depletion of NLRP3 expression, or that of other inflammasome components (such as ASC or CASP1) enhanced the infectivity of macrophages by HIV-1, revealed that all proteins belonging to the NLRP3 inflammasome function together as novel restriction mechanism for HIV-1. In sharp contrast to other identified restriction factors that impair viral infection at post-entry steps³², however, NLRP3, ASC and CASP1 limit HIV-1 infection by interfering with viral fusion and entry. Moreover, we showed that NLRP3 inflammasome activators could reduce the infectability of HIV-1 target cells, demonstrating that the NLRP3 inflammasome is an inducible restriction complex for HIV-1 that can be targeted for the prevention and the treatment of HIV-1 induced pathologies. Altogether, our data provide a molecular explanation of how M1 polarization of macrophages alters their susceptibility to HIV-1 infection³³ and how HIV-1 hijacked innate immunity to accomplish its viral life cycle. Future studies should explore the possibility to modulate the physical and functional NLRP3/P2Y2 interaction with the scope of reducing HIV-1 infection, but also of treating human diseases that are associated with excessive activation of NLRP3 inflammasome (such cryoporin associated periodic syndromes¹⁵ or arthritis rheumatoid³⁴).

### REFERENCES:

Throughout this application, various references describe the state of the art to which this invention pertains. The disclosures of these references are hereby incorporated by reference into the present disclosure.
1 Finlay, B. B. & Hancock, R. E. Can innate immunity be enhanced to treat microbial infections? Nature reviews. Microbiology 2, 497-504, doi:10.1038/nrmicro908 (2004).
2 McMichael, A. J. & Koff, W. C. Vaccines that stimulate T cell immunity to HIV-1: the next step. Nature immunology 15, 319-322, doi:10.1038/ni.2844 (2014).
3 Iwasaki, A. Innate immune recognition of HIV-1. Immunity 37, 389-398, doi:10.1016/j.immuni.2012.08.011 (2012).
4 Cox, A. L. & Siliciano, R. F. Making sense of HIV innate sensing. Immunity 39, 998-1000, doi:10.1016/j.immuni.2013.11.014 (2013).
5 Towers, G. J. & Noursadeghi, M. Interactions between HIV-1 and the cell-autonomous innate immune system. Cell host & microbe 16, 10-18, doi:10.1016/j.chom.2014.06.009 (2014).
6 Silvin, A. & Manel, N. Innate immune sensing of HIV infection. Current opinion in immunology 32C, 54-60, doi:10.1016/j.coi.2014.12.003 (2015).
7 Doitsh, G. et al. Cell death by pyroptosis drives CD4 T-cell depletion in HIV-1 infection. Nature 505, 509-514, doi:10.1038/nature12940 (2014).
8 Monroe, K. M. et al. IFI16 DNA sensor is required for death of lymphoid CD4 T cells abortively infected with HIV. Science 343, 428-432, doi:10.1126/science.1243640 (2014).
9 Doitsh, G. et al. Abortive HIV infection mediates CD4 T cell depletion and inflammation in human lymphoid tissue. Cell 143, 789-801, doi:10.1016/j.cell.2010.11.001 (2010).
10 Seror, C. et al. Extracellular ATP acts on P2Y2 purinergic receptors to facilitate HIV-1 infection. The Journal of experimental medicine 208, 1823-1834, doi:10.1084/jem.20101805 (2011).
11 Swartz, T. H., Esposito, A. M., Durham, N. D., Hartmann, B. M. & Chen, B. K. P2X-selective purinergic antagonists are strong inhibitors of HIV-1 fusion during both cell-to-cell and cell-free infection. Journal of virology 88, 11504-11515, doi:10.1128/JVI.01158-14 (2014).
12 Hazleton, J. E., Berman, J. W. & Eugenin, E. A. Purinergic Receptors Are Required for HIV-1 Infection of Primary Human Macrophages. J Immunol 188, 4488-4495, doi:10.4049/jimmunol.1102482 (2012).
13 Paoletti, A. et al. Multifaceted roles of purinergic receptors in viral infection. Microbes and infection / Institut Pasteur 14, 1278-1283, doi:10.1016/j.micinf.2012.05.010 (2012).
14 Sutterwala, F. S., Haasken, S. & Cassel, S. L. Mechanism of NLRP3 inflammasome activation. Annals of the New York Academy of Sciences 1319, 82-95, doi:10.1111/nyas.12458 (2014).
15 Agostini, L. et al. NALP3 forms an IL-1beta-processing inflammasome with increased activity in Muckle-Wells autoinflammatory disorder. Immunity 20, 319-325 (2004).
16 Hari, A. et al. Activation of NLRP3 inflammasome by crystalline structures via cell surface contact. Scientific reports 4, 7281, doi:10.1038/srep07281 (2014).
17 Elliott, M. R. et al. Nucleotides released by apoptotic cells act as a findme signal to promote phagocytic clearance. Nature 461, 282-286, doi:10.1038/nature08296 (2009).
18 Kronlage, M. et al. Autocrine purinergic receptor signaling is essential for macrophage chemotaxis. Science signaling 3, ra55, doi:10.1126/scisignal.2000588 (2010).
19 Murray, P. J. et al. Macrophage activation and polarization: nomenclature and experimental guidelines. Immunity 41, 14-20, doi:10.1016/j.immuni.2014.06.008 (2014).
20 Krausgruber, T. et al. IRF5 promotes inflammatory macrophage polarization and TH1-TH17 responses. Nature immunology 12, 231-238, doi:10.1038/ni.1990 (2011).
21 Bergsbaken, T., Fink, S. L. & Cookson, B. T. Pyroptosis: host cell death and inflammation. Nature reviews. Microbiology 7, 99-109, doi:10.1038/nrmicro2070 (2009).
22 Dostert, C. et al. Innate immune activation through Nalp3 inflammasome sensing of asbestos and silica. Science 320, 674-677, doi:10.1126/science.1156995 (2008).
23 Tschopp, J. & Schroder, K. NLRP3 inflammasome activation: The convergence of multiple signalling pathways on ROS production? Nature reviews. Immunology 10, 210-215, doi:10.1038/nri2725 (2010).
24 Youle, R. J. & Narendra, D. P. Mechanisms of mitophagy. Nature reviews. Molecular cell biology 12, 9-14, doi:10.1038/nrm3028 (2011).
25 Zhou, R., Yazdi, A. S., Menu, P. & Tschopp, J. A role for mitochondria in NLRP3 inflammasome activation. Nature 469, 221-225, doi:10.1038/nature09663 (2011).
26 Alers, S., Loffler, A. S., Wesselborg, S. & Stork, B. Role of AMPK-mTOR-Ulk1/2 in the regulation of autophagy: cross talk, shortcuts, and feedbacks. Molecular and cellular biology 32, 2-11, doi:10.1128/MCB.06159-11 (2012).
27 Okigaki, M. et al. Pyk2 regulates multiple signaling events crucial for macrophage morphology and migration. Proceedings of the National Academy of Sciences of the United States of America 100, 10740-10745, doi:10.1073/pnas.1834348100 (2003).
28 Py, B. F., Kim, M. S., Vakifahmetoglu-Norberg, H. & Yuan, J. Deubiquitination of NLRP3 by BRCC3 critically regulates inflammasome activity. Molecular cell 49, 331-338, doi:10.1016/j.molcel.2012.11.009 (2013).
29 Kankkunen, P. et al. Trichothecene mycotoxins activate NLRP3 inflammasome through a P2X7 receptor and Src tyrosine kinase dependent pathway. Human immunology 75, 134-140, doi:10.1016/j.humimm.2013.11.010 (2014).
30 Sandilands, E. et al. Autophagic targeting of Src promotes cancer cell survival following reduced FAK signalling. Nature cell biology 14, 51-60, doi:10.1038/ncb2386 (2012).
31 Reiser, J. et al. Transduction of nondividing cells using pseudotyped defective high-titer HIV type 1 particles. Proceedings of the National Academy of Sciences of the United States of America 93, 15266-15271 (1996).
32 Harris, R. S., Hultquist, J. F. & Evans, D. T. The restriction factors of human immunodeficiency virus. The Journal of biological chemistry 287, 40875-40883, doi:10.1074/jbc.R112.416925 (2012).
33 Cassol, E., Cassetta, L., Alfano, M. & Poli, G. Macrophage polarization and HIV-1 infection. Journal of leukocyte biology 87, 599-608, doi:10.1189/jlb.1009673 (2010).
34 Vande Walle, L. et al. Negative regulation of the NLRP3 inflammasome by A20 protects against arthritis. Nature 512, 69-73, doi:10.1038/nature13322 (2014).
35 Shi, C. S. et al. Activation of autophagy by inflammatory signals limits IL-1beta production by targeting ubiquitinated inflammasomes for destruction. Nature immunology 13, 255-263, doi:10.1038/ni.2215 (2012).
36 Allouch, A. et al. p21-mediated RNR2 repression restricts HIV-1 replication in macrophages by inhibiting dNTP biosynthesis pathway. Proceedings of the National Academy of Sciences of the United States of America 110, E3997-4006, doi:10.1073/pnas.1306719110 (2013).
37 He, Y. et al. 3,4-methylenedioxy-beta-nitrostyrene inhibits NLRP3 inflammasome activation by blocking assembly of the inflammasome. The Journal of biological chemistry 289, 1142-1150, doi:10.1074/jbc.M113.515080 (2014).
38 Seye, C. I., Yu, N., Gonzalez, F. A., Erb, L. & Weisman, G. A. The P2Y2 nucleotide receptor mediates vascular cell adhesion molecule-1 expression through interaction with VEGF receptor-2 (KDR/Flk-1). The Journal of biological chemistry 279, 35679-35686, doi:10.1074/jbc.M401799200 (2004).
39 Perfettini, J. L. et al. NF-kappaB and p53 are the dominant apoptosis-inducing transcription factors elicited by the HIV-1 envelope. The Journal of experimental medicine 199, 629-640, doi:10.1084/jem.20031216 (2004).
40 Perfettini, J. L. et al. Essential role of p53 phosphorylation by p38 MAPK in apoptosis induction by the HIV-1 envelope. The Journal of experimental medicine 201, 279-289, doi:10.1084/jem.20041502 (2005).
41 Delelis, O. et al. The G140S mutation in HIV integrases from raltegravir-resistant patients rescues catalytic defect due to the resistance Q148H mutation. Nucleic acids research 37, 1193-1201, doi:10.1093/nar/gkn1050 (2009).
42 Melki, M. T., Saidi, H., Dufour, A., Olivo-Marin, J. C. & Gougeon, M. L. Escape of HIV-1-infected dendritic cells from TRAIL-mediated NK cell cytotoxicity during NK-DC cross-talk--a pivotal role of HMGB1. PLoS pathogens 6, e1000862, doi:10.1371/journal.ppat.1000862 (2010).
43 Homolya, L., Watt, W. C., Lazarowski, E. R., Koller, B. H. & Boucher, R. C. Nucleotide-regulated calcium signaling in lung fibroblasts and epithelial cells from normal and P2Y(2) receptor (-/-) mice. The Journal of biological chemistry 274, 26454-26460 (1999).
44 Johnston, L. K., Rims, C. R., Gill, S. E., McGuire, J. K. & Manicone, A. M. Pulmonary macrophage subpopulations in the induction and resolution of acute lung injury. American journal of respiratory cell and molecular biology 47, 417-426, doi:10.1165/rcmb.2012-00900C (2012).
45 Dioszeghy, V. et al. Changes in soluble factor-mediated CD8+ cell-derived antiviral activity in cynomolgus macaques infected with simian immunodeficiency virus SIVmac251: relationship to biological markers of progression. Journal of virology 80, 236-245, doi:10.1128/JVI.80.1.236-245.2006 (2006).

## Claims

1. An *in vitro* method for promoting secretion of an inflammatory cytokine selected from the group consisting of IL-lbeta, TNF, IL-12, and IL-23 by macrophages, comprising contacting said macrophages with an inhibitor of P2Y2 receptor activity or expression.

2. The *in vitro* method of claim 1, wherein said macrophages have been obtained from a subject suffering from cancer, preferably from a blood-born cancer.

3. The *in vitro* method of claim 1 or 2, wherein said inhibitor causes an increase in the M1/M2 polarization ratio.

4. The *in vitro* method of claims 1 to 3, wherein said inhibitor is a small organic molecule such as diphosphoric 5-(2,4-dioxo-3,4-dihydropyrimidin-1(2H)-yl)pentylphosphonic anhydre or 4-phenyl-amino-4-(2-methoxyphenyl)-2-sulfoanthraquinone.

5. The *in vitro* method of claims 1 to 4, wherein said inhibitor is an antibody, an aptamer, a ribozyme or an antisense oligonucleotide.

6. An inhibitor of P2Y2 receptor activity or expression, for use for reducing macrophage M2 polarization in a subject suffering from cancer.

7. The inhibitor for use according to claim 6, wherein said cancer is a blood cell cancer, such as leukemia or lymphoma.

8. The inhibitor for use according to claim 6, wherein said cancer is selected in the group consisting of: malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

9. The inhibitor for use according to claim 6, wherein it is a small organic molecule such as diphosphoric 5-(2,4-dioxo-3,4-dihydropyrimidin-1(2H)-yl)pentylphosphonic anhydre or 4-phenyl-amino-4-(2-methoxyphenyl)-2-sulfoanthraquinone.

10. The inhibitor for use according to claim 6, wherein it is an antibody, an aptamer, a ribozyme or an antisense oligonucleotide.
